(19) Europäisches Patentamt / European Patent Office / Office européen des brevets

(11) **EP 4 768 027 A1**

(12) **EUROPEAN PATENT APPLICATION**
published in accordance with Art. 153(4) EPC


(43) Date of publication:
**01.07.2026 Bulletin 2026/27**

(21) Application number: **24856541.8**

(22) Date of filing: **23.08.2024**

(51) International Patent Classification (IPC):
***A61K 31/437*** (2006.01) ***A61K 31/568*** (2006.01)
***A61K 31/4155*** (2006.01) ***A61K 31/4439*** (2006.01)
***A61K 45/00*** (2006.01) ***A61P 5/28*** (2006.01)
***A61P 13/08*** (2006.01) ***A61P 35/00*** (2006.01)
***A61P 43/00*** (2006.01)

(52) Cooperative Patent Classification (CPC):
**A61K 31/4155; A61K 31/437; A61K 31/4439; A61K 31/568; A61K 45/00; A61P 5/28; A61P 13/08; A61P 35/00; A61P 43/00**

(86) International application number:
**PCT/JP2024/029998**

(87) International publication number:
**WO 2025/041853 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.08.2023 JP 2023136438**



(71) Applicant: **Taiho Pharmaceutical Co., Ltd.**
**Chiyoda-ku, Tokyo 101-8444 (JP)**

(72) Inventors:
- **MINAMIGUCHI, Kazuhisa**
  **Tsukuba-shi, Ibaraki 300-2611 (JP)**
- **MURAOKA, Hiromi**
  **Tsukuba-shi, Ibaraki 300-2611 (JP)**
- **SHINOZAKI, Atsuki**
  **Tokyo 101-8444 (JP)**

(74) Representative: **Wächtershäuser & Hartz Patentanwaltspartnerschaft mbB**
**Weinstraße 8**
**80333 München (DE)**


(54) **CANCER COMBINATION THERAPY USING AZA BICYCLIC COMPOUND AND ANTIANDROGEN AGENT**


(57) Provided is a novel method for treating CRPC, particularly, CRPC which has acquired resistance to an antiandrogen agent. The present invention relates to an antitumor agent wherein 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof and an antiandrogen agent are administered in combination.


Fig. 1

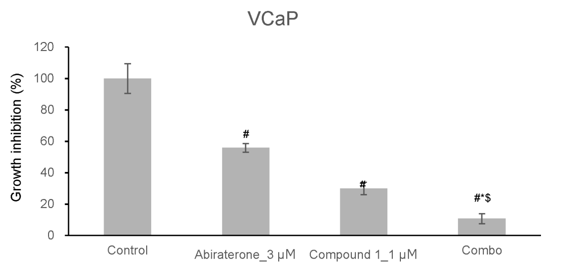


#: $p<0.05$ with Aspin-Welch's *t*-test as compared with the Control group.

*: $p<0.05$ with Aspin-Welch's *t*-test as compared with the Abiraterone group.

$: $p<0.05$ with Aspin-Welch's *t*-test as compared with the Compound 1 control group.

Data are presented as mean ± standard error.

EP 4 768 027 A1

## Description

Field of the Invention

The present invention relates to combination therapy using 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof and an antiandrogen agent for a cancer patient.

Background of the Invention

Prostate cancer is the most frequent cancer which affects males in Europe and the United States and is the second leading cause of cancer death. In Japan as well, the number of prostate cancer patients is increasing every year along with the westernization of diet and population aging. In general, the proliferation of prostate cancer cells is stimulated by androgen. Therefore, androgen ablative therapy such as surgical or chemical castration is mainly used in the treatment of unresectable advanced prostate cancer. The androgen ablative therapy decreases an androgen level in the body and exhibits a therapeutic effect such as a decreased prostate-specific antigen (PSA) level or tumor regression in many patients. However, disease progression or recurrence may be found in many patients within several years, though the androgen level is a castration level. Such recurrent prostate cancer is called castration resistant prostate cancer (CRPC). For example, androgen receptor (AR) gene amplification and overexpression have been reported for CRPC patients (Non Patent Literatures 1 to 3). Castration resistant prostate cancer is considered to exhibit high sensitivity even to an ultralow concentration of androgen due to the overexpression of AR.

Antiandrogen agents such as abiraterone, enzalutamide, darolutamide, and apalutamide, and taxane-based antitumor agents such as docetaxel and cabazitaxel have been approved as therapeutic drugs for CRPC and widely used in clinical practice. However, cross-resistance due to the mechanisms such as AR gene amplification has been reported for the antiandrogen agents, and the efficacy of the antiandrogen agents for use as the second or later agent is known to be limited (Non Patent Literature 4). The taxane-based antitumor agents exhibit a high incidence rate of hematotoxicity (neutropenia and anemia). Since a majority of prostate cancer patients are elderly, careful administration is required, and such toxicity is considered to present a clinical problem (Non Patent Literature 5). Thus, there is a demand for a novel method for treating CRPC, particularly, CRPC which has acquired resistance to an antiandrogen agent.

3-Ethyl-4-{4-[4-(1-methyl-1H-pyrazol-4-yl)-1H-imidazol-1-yl]-3-(propan-2-yl)-1H-pyrazolo[3,4-b]pyridin-1-yl} benzamide (generic name: pimitespib; hereinafter, also referred to as "compound 1") is a compound which exhibits an antitumor effect through excellent HSP90 inhibitory activity (Patent Literature 1).

HSP90, a member of a group of proteins called molecular chaperones, is known to function in many aspects such as the promotion or retention of formation of functional structures of a wide range of client proteins, the promotion of correct association, the suppression of unnecessary aggregation, protection from decomposition, and the promotion of secretion (Non Patent Literature 6). Because of the physiological functions of HSP90, an HSP90 inhibitor has the feature that the HSP90 inhibitor can simultaneously inhibit a plurality of signal transduction pathways involved in the survival and growth of a cancer. Therefore, the HSP90 inhibitor has heretofore been expected to serve as a drug having a broad range of and effective antitumor effect (Non Patent Literature 7).

The compound 1 has been approved as a medicament having efficacy or an effect on "gastrointestinal stromal tumor which has progressed after cancer chemotherapy" in Japan and used in clinical practice (product name: Jeselhy(R)). Patent Literatures 2 to 6 disclose use of the compound 1 and use of the compound 1 in combination with other anticancer agents.

Since AR is one of the client proteins of HSP90, combined use of an HSP90 inhibitor such as NVP-AUY922 and an antiandrogen agent has been conventionally expected to a treatment for CRPC from the viewpoint of the mechanism of action (Non Patent Literature 8). However, it has been reported in recent years that combined use of AT13387 (Onalespib), one of the HSP90 inhibitors, and abiraterone exhibited no sufficient therapeutic effect in clinical trials conducted targeting CRPC patients (Non Patent Literature 9).

Citation List

Patent Literature

Patent Literature 1: WO 2011/004610
Patent Literature 2: WO 2015/046498
Patent Literature 3: WO 2019/004417

Patent Literature 4: WO 2019/054465
Patent Literature 5: WO 2019/221086
Patent Literature 6: WO 2021/025065

Non Patent Literature

Non Patent Literature 1: J Clin Oncol. 2012; 30 (6): 644-646
Non Patent Literature 2: Oncotarget. 2016; 7 (39): 64447-64470
Non Patent Literature 3: Nat Med. 2016; 22 (4): 369-378
Non Patent Literature 4: Lancet Oncol. 2019; 20 (12): 1730-1739
Non Patent Literature 5: Cancer Med. 2016; 5 (2): 182-191
Non Patent Literature 6: Nat Rev Cancer. 2005; 5 (10): 761-772
Non Patent Literature 7: Trends Mol Med. 2004; 10 (6): 283-290
Non Patent Literature 8: Endocr Relat Cancer. 2015; 22 (5): 805-818
Non Patent Literature 9: Clin Cancer Res. 2019; 25 (15): 4624-4633

Summary of the Invention

Technical Problem

An object of the present invention is to provide a novel method for treating CRPC, particularly, CRPC which has acquired resistance to an antiandrogen agent.

Solution to Problem

The present inventors have conducted diligent studies to attain the object and consequently found that a combination of compound 1 and an antiandrogen agent markedly enhances an antitumor effect on CRPC without augmenting adverse reactions.
Specifically, the present invention provides the following [1] to [72].

[1] An antitumor agent wherein compound 1 or a salt thereof and an antiandrogen agent are administered in combination.
[2] The antitumor agent according to [1], in which the antiandrogen agent is one or more members selected from the group consisting of abiraterone, enzalutamide, darolutamide, and apalutamide.
[3] The antitumor agent according to [1], in which the antiandrogen agent is enzalutamide.
[4] The antitumor agent according to any one of [1] to [3] which is an antitumor agent for treating prostate cancer.
[5] The antitumor agent according to any one of [1] to [3] which is an antitumor agent for treating castration resistant prostate cancer.
[6] The antitumor agent according to any one of [1] to [5], in which the compound 1 or the salt thereof and the antiandrogen agent are administered concurrently or separately in a staggered manner to a cancer patient.
[7] The antitumor agent according to any one of [1] to [6], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.
[8] The antitumor agent according to any one of [1] to [6], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.
[9] The antitumor agent according to any one of [1] to [8], in which a taxane-based antitumor agent, a prostate-specific membrane antigen (PSMA)-targeting therapeutic drug, or a PARP inhibitor is further administered in combination therewith.
[10] An antitumor effect enhancer for an antiandrogen agent, comprising compound 1 or a salt thereof as an active ingredient.
[11] The antitumor effect enhancer according to [10], in which the antiandrogen agent is one or more members selected from the group consisting of abiraterone, enzalutamide, darolutamide, and apalutamide.
[12] The antitumor effect enhancer according to [10], in which the antiandrogen agent is enzalutamide.

[13] The antitumor effect enhancer according to any one of [10] to [12] which is an antitumor agent for treating prostate cancer.

[14] The antitumor effect enhancer according to any one of [10] to [12] which is an antitumor agent for treating castration resistant prostate cancer.

[15] The antitumor effect enhancer according to any one of [10] to [14], in which the compound 1 or the salt thereof and the antiandrogen agent are administered concurrently or separately in a staggered manner to a cancer patient.

[16] The antitumor effect enhancer according to any one of [10] to [15], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.

[17] The antitumor effect enhancer according to any one of [10] to [15], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.

[18] The antitumor effect enhancer according to any one of [10] to [15], in which a taxane-based antitumor agent, a prostate-specific membrane antigen (PSMA)-targeting therapeutic drug, or a PARP inhibitor is further administered in combination therewith.

[19] An antitumor agent comprising compound 1 or a salt thereof as an active ingredient, in which the antitumor agent is administered in combination with an antiandrogen agent.

[20] The antitumor agent according to [19], in which the antiandrogen agent is one or more members selected from the group consisting of abiraterone, enzalutamide, darolutamide, and apalutamide.

[21] The antitumor agent according to [19], in which the antiandrogen agent is enzalutamide.

[22] The antitumor agent according to any one of [19] to [21] which is an antitumor agent for treating prostate cancer.

[23] The antitumor agent according to any one of [19] to [21] which is an antitumor agent for treating castration resistant prostate cancer.

[24] The antitumor agent according to any one of [19] to [23], in which the compound 1 or the salt thereof and the antiandrogen agent are administered concurrently or separately in a staggered manner to a cancer patient.

[25] The antitumor agent according to any one of [19] to [24], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.

[26] The antitumor agent according to any one of [19] to [24], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.

[27] The antitumor agent according to any one of [19] to [26], in which a taxane-based antitumor agent, a prostate-specific membrane antigen (PSMA)-targeting therapeutic drug, or a PARP inhibitor is further administered in combination therewith.

[28] An antitumor agent comprising a combination of compound 1 or a salt thereof and an antiandrogen agent.

[29] The antitumor agent according to [28], in which the antiandrogen agent is one or more members selected from the group consisting of abiraterone, enzalutamide, darolutamide, and apalutamide.

[30] The antitumor agent according to [28], in which the antiandrogen agent is enzalutamide.

[31] The antitumor agent according to any one of [28] to [30] which is an antitumor agent for treating prostate cancer.

[32] The antitumor agent according to any one of [28] to [30] which is an antitumor agent for treating castration resistant prostate cancer.

[33] The antitumor agent according to any one of [28] to [32], in which the compound 1 or the salt thereof and the antiandrogen agent are administered concurrently or separately in a staggered manner to a cancer patient.

[34] The antitumor agent according to any one of [28] to [33], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.

[35] The antitumor agent according to any one of [28] to [33], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.

[36] The antitumor agent according to any one of [28] to [35], in which a taxane-based antitumor agent, a prostate-specific membrane antigen (PSMA)-targeting therapeutic drug, or a PARP inhibitor is further administered in combination therewith.

[37] A method for treating a cancer, comprising the step of administering a therapeutically effective amount of compound 1 or a salt thereof and a therapeutically effective amount of an antiandrogen agent to a cancer patient.
[38] The method for treating a cancer according to [37], in which the antiandrogen agent is one or more members selected from the group consisting of abiraterone, enzalutamide, darolutamide, and apalutamide.
[39] The method for treating a cancer according to [37], in which the antiandrogen agent is enzalutamide.
[40] The method for treating a cancer according to any one of [37] to [39], in which the cancer is prostate cancer.
[41] The method for treating a cancer according to any one of [37] to [39], in which the cancer is castration resistant prostate cancer.
[42] The method for treating a cancer according to any one of [37] to [41], in which the compound 1 or the salt thereof and the antiandrogen agent are administered concurrently or separately in a staggered manner to the cancer patient.
[43] The method for treating a cancer according to any one of [37] to [42], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.
[44] The method for treating a cancer according to any one of [37] to [42], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.
[45] The method for treating a cancer according to any one of [37] to [44], in which a taxane-based antitumor agent, a prostate-specific membrane antigen (PSMA)-targeting therapeutic drug, or a PARP inhibitor is further administered in combination therewith.
[46] Use of compound 1 or a salt thereof for the treatment of a cancer, in which the compound 1 or the salt thereof is administered in combination with an antiandrogen agent to a cancer patient.
[47] The use according to [46], in which the antiandrogen agent is one or more members selected from the group consisting of abiraterone, enzalutamide, darolutamide, and apalutamide.
[48] The use according to [46], in which the antiandrogen agent is enzalutamide.
[49] The use according to any one of [46] to [48], in which the cancer is prostate cancer.
[50] The use according to any one of [46] to [48], in which the cancer is castration resistant prostate cancer.
[51] The use according to any one of [46] to [50], in which the compound 1 or the salt thereof and the antiandrogen agent are administered concurrently or separately in a staggered manner to the cancer patient.
[52] The use according to any one of [46] to [51], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.
[53] The use according to any one of [46] to [51], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.
[54] The use according to any one of [46] to [53], in which a taxane-based antitumor agent, a prostate-specific membrane antigen (PSMA)-targeting therapeutic drug, or a PARP inhibitor is further administered in combination therewith.
[55] Use of compound 1 or a salt thereof for the production of an antitumor agent, in which the antitumor agent is administered in combination with an antiandrogen agent to a cancer patient.
[56] The use according to [55], in which the antiandrogen agent is one or more members selected from the group consisting of abiraterone, enzalutamide, darolutamide, and apalutamide.
[57] The use according to [55], in which the antiandrogen agent is enzalutamide.
[58] The use according to any one of [55] to [57], in which the cancer is prostate cancer.
[59] The use according to any one of [55] to [57], in which the cancer is castration resistant prostate cancer.
[60] The use according to any one of [55] to [59], in which the compound 1 or the salt thereof and the antiandrogen agent are administered concurrently or separately in a staggered manner to the cancer patient.
[61] The use according to any one of [55] to [60], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.
[62] The use according to any one of [55] to [60], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.

[63] The use according to any one of [55] to [62], in which a taxane-based antitumor agent, a prostate-specific membrane antigen (PSMA)-targeting therapeutic drug, or a PARP inhibitor is further administered in combination therewith.

[64] Compound 1 or a salt thereof for use in the treatment of a cancer, in which the compound 1 or the salt thereof is administered in combination with an antiandrogen agent to a cancer patient.

[65] The compound 1 or the salt thereof according to [64], in which the antiandrogen agent is one or more members selected from the group consisting of abiraterone, enzalutamide, darolutamide, and apalutamide.

[66] The compound 1 or the salt thereof according to [64], in which the antiandrogen agent is enzalutamide.

[67] The compound 1 or the salt thereof according to any one of [64] to [66], in which the cancer is prostate cancer.

[68] The compound 1 or the salt thereof according to any one of [64] to [66], in which the cancer is castration resistant prostate cancer.

[69] The compound 1 or the salt thereof according to any one of [64] to [68], in which the compound 1 or the salt thereof and the antiandrogen agent are administered concurrently or separately in a staggered manner to the cancer patient.

[70] The compound 1 or the salt thereof according to any one of [64] to [69], in which the compound 1 or the salt thereof is continuously administered at 120 mg as the compound 1 once a day for 5 days, followed by a drug holiday of 2 days, and enzalutamide is continuously administered at 160 mg once a day for 7 days, and this 7-day administration schedule is performed once or two or more repeated times.

[71] The compound 1 or the salt thereof according to any one of [64] to [69], in which the compound 1 or the salt thereof is continuously administered at 160 mg as the compound 1 once a day for 5 days, followed by a drug holiday of 2 days, and enzalutamide is continuously administered at 160 mg once a day for 7 days, and this 7-day administration schedule is performed once or two or more repeated times.

[72] The compound 1 or the salt thereof according to any one of [64] to [71], in which a taxane-based antitumor agent, a prostate-specific membrane antigen (PSMA)-targeting therapeutic drug, or a PARP inhibitor is further administered in combination therewith.

Advantageous Effects of Invention

The antitumor agent of the present invention is capable of performing cancer treatment which exerts a high antitumor effect (e.g., the prolongation of a survival time (particularly, the prolongation of an overall survival, radiographic progression-free survival, or the like), tumor reduction, and delayed tumor growth) while suppressing the manifestation of adverse reactions. Accordingly, the antitumor agent of the present invention brings about long-term survival of cancer patients. The antitumor agent of the present invention experts a high antitumor effect on, particularly, prostate cancer, HSPC, CRPC, and CRPC which has acquired resistance to an antiandrogen agent.

Brief Description of Drawings

Figure 1 illustrates results of compound 1 + abiraterone *in vitro* combination analysis.

Figure 2 illustrates results of compound 1 + enzalutamide *in vitro* combination analysis.

Figure 3-1 illustrates results of compound 1 + darolutamide *in vitro* combination analysis.

Figure 3-2 illustrates results of compound 1 + darolutamide *in vitro* combination analysis.

Figure 4 illustrates results about a compound 1 + enzalutamide combination in a castration resistant model in which a prostate cancer line LNCaP was transplanted.

Figure 5 illustrates results about a compound 1 + enzalutamide combination in a castration resistant model in which a prostate cancer line VCaP was transplanted.

Figure 6 illustrates results about a compound 1 + darolutamide combination in a castration resistant model in which a prostate cancer line LNCaP was transplanted.

Figure 7 illustrates results about a compound 1 + abiraterone combination in a castration resistant model in which a prostate cancer line VCaP was transplanted.

Figure 8 illustrates the summary of test design of combination therapy with pimitespib and enzalutamide.

Description of Embodiments

The present invention relates to an antitumor agent, an antitumor effect enhancer, a kit preparation, and use of these drugs, a method for treating tumor, and a method for enhancing an antitumor effect, in which compound 1 or a salt thereof and an antiandrogen agent are administered in combination.

[Compound 1 or salt thereof]

In the present invention, the compound 1 is 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide, also called pimitespib as a generic name, and is a compound having the following structure:

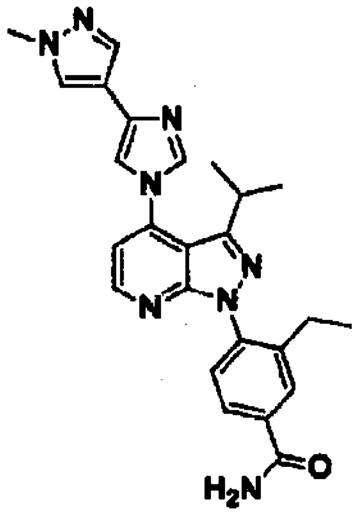

When the compound 1 has isomers such as optical isomers, stereoisomers, rotational isomers, or tautomers, any mixture of the isomers is also encompassed by the compound 1 unless otherwise specified.
The compound 1 or the salt thereof may be a solvate (e.g., a hydrate) or may be a non-solvate. In the present invention, both the solvate and the non-solvate are encompassed by the "compound 1 or salt thereof".
The "salt" of the compound 1 means a pharmaceutically acceptable salt. Specific examples thereof can include: acid-addition salts with inorganic acids such as hydrochloric acid, hydrobromic acid, hydroiodic acid, sulfuric acid, nitric acid, and phosphoric acid or organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, p-toluenesulfonic acid, and glutamic acid; salts with inorganic bases such as sodium, potassium, magnesium, calcium, and aluminum, organic bases such as methylamine, ethylamine, meglumine, and ethanolamine, or basic amino acids such as lysine, arginine, and ornithine; and ammonium salts.
The compound 1 or the salt thereof is a known compound and can be synthesized in accordance with, for example, a method described in Patent Literature 1 (WO 2011/004610). Alternatively, a product sold in Japan (Jeselhy(R)) may be used.

[Antiandrogen agent]

As described in Examples mentioned later, the compound 1 of the present invention or the salt thereof synergistically enhances an antitumor effect when administered in combination with an antiandrogen agent.
The "antiandrogen agent" according to the present invention is a drug which exhibits only antagonistic activity without exhibiting agonistic activity against androgen receptor (AR). The form of the drug is not particularly limited and may be a small-molecule drug or may be an antibody drug, a nucleic acid drug, or the like. Specific examples of the antiandrogen agent according to the present invention include abiraterone, enzalutamide, darolutamide, and apalutamide. The antiandrogen agent is preferably one or more members selected from the group consisting of abiraterone, enzalutamide, darolutamide, and apalutamide, more preferably one or more members selected from the group consisting of abiraterone, enzalutamide, and darolutamide, more preferably one or more members selected from the group consisting of abiraterone and enzalutamide, particularly preferably enzalutamide, from the viewpoint of a synergistic effect with the compound 1 of the present invention or the salt thereof. Commercially available products may be used as these antiandrogen agents, or these antiandrogen agents may be synthesized by well-known common production methods.
In addition to the antiandrogen agent, an additional antitumor agent may be further administered in combination therewith. In this case, the additional antitumor agent is not particularly limited as long as the antitumor agent does not inhibit a combinatorial effect of the compound 1 or the salt thereof and the antiandrogen agent. Examples thereof may include taxane-based antitumor agents, prostate-specific membrane antigen (PSMA)-targeting therapeutic drugs, and PARP inhibitors. Examples of the taxane-based antitumor agent include docetaxel and cabazitaxel. Examples of the

PSMA-targeting therapeutic drug include lutetium (177Lu) vipivotide tetraxetan (Pluvicto). Examples of the PARP inhibitor include olaparib, rucaparib, talazoparib, niraparib, and veliparib.

[Target disease]

In the present invention, the target cancer is not particularly limited as long as the compound 1 or the salt thereof and the antiandrogen agent exert a synergistic effect thereon. Specific examples thereof include head and neck cancer, digestive system cancer (esophageal cancer, stomach cancer, duodenal cancer, liver cancer, biliary tract cancer (gallbladder/bile duct cancer, and the like), pancreatic cancer, small intestinal cancer, large intestinal cancer (colorectal cancer, colon cancer, rectal cancer, and the like), and the like), lung cancer (non-small cell lung cancer, small-cell lung cancer, and the like), breast cancer, ovarian cancer, uterine cancer (cervical cancer, endometrial cancer, and the like), kidney cancer, urinary bladder cancer, prostate cancer, skin cancer (malignant melanoma, epidermal cancer, and the like), blood cancer (multiple myeloma, acute myeloid leukemia, and the like), and sarcoma (osteosarcoma, soft tissue sarcoma, uterine sarcoma, gastrointestinal stromal tumor, and the like).

Among these, prostate cancer is preferred from the viewpoint of a combinatorial effect of the compound 1 or the salt thereof and the antiandrogen agent. The cancer is further more preferably hormone-sensitive prostate cancer (hereinafter, also referred to as "HSPC"; also called castration-sensitive prostate cancer (CSPC)) or castration resistant prostate cancer (CRPC), further more preferably castration resistant prostate cancer (CRPC), further more preferably castration resistant prostate cancer which has acquired resistance to an antiandrogen agent. Herein, the prostate cancer is not particularly limited by the presence or absence of distant metastasis and includes, for example, metastatic castration resistant prostate cancer, nonmetastatic castration resistant prostate cancer, metastatic hormone-sensitive prostate cancer, and nonmetastatic hormone-sensitive prostate cancer. Herein, the "castration resistant prostate cancer which has acquired resistance to an antiandrogen agent" is castration resistant prostate cancer which has progressed after cancer chemotherapy with the antiandrogen agent. Herein, examples of the "antiandrogen agent" can include antiandrogen agents described above. Abiraterone is preferred.

[Dosage and administration]

In the present invention, the daily dose of the compound 1 or the salt thereof on an administration day is preferably from 50 to 150%, more preferably from 50 to 125%, more preferably from 50 to 100%, more preferably from 75 to 100%, particularly preferably 100%, of the recommended dose when the compound 1 or the salt thereof is administered alone from the viewpoint of an enhancing effect on an antitumor effect.

Specifically, the recommended dose when the compound 1 or the salt thereof administered alone is usually 160 mg/day as the compound 1 for an adult. In a usual administration, the compound 1 or the salt thereof is administered at 160 mg as the compound 1 once a day. If necessary, the dose may be increased or decreased. In the present invention, the daily dose is preferably from 80 to 240 mg/day, more preferably from 80 to 200 mg/day, more preferably from 80 to 160 mg/day, more preferably from 120 to 160 mg/day, particularly preferably from 160 mg/day, as the compound 1. Specifically, the daily dose is preferably 80 mg/day, 120 mg/day, 160 mg/day, 200 mg/day, or 240 mg/day, further more preferably 80 mg/day, 120 mg/day, or 160 mg/day, further more preferably 120 mg/day or 160 mg/day, particularly preferably 160 mg/day, as the compound 1.

In the present invention, the daily dose on an administration day of the antiandrogen agent is preferably from 50 to 150%, more preferably from 50 to 100%, more preferably from 75 to 100%, particularly preferably 100%, of the recommended dose of the antiandrogen agent administered alone from the viewpoint of an enhancing effect on an antitumor effect.

Specifically, the recommended dose of abiraterone administered alone is usually 1000 mg/day as abiraterone acetate for an adult. In a usual administration, abiraterone is administered at 1000 mg as abiraterone acetate once a day. If necessary, the dose may be increased or decreased. In the present invention, the daily dose is preferably from 500 to 1500 mg, more preferably from 500 to 1000 mg, more preferably from 750 to 1000 mg, particularly preferably 1000 mg, as abiraterone acetate.

The recommended dose of enzalutamide administered alone is usually 160 mg/day as abiraterone acetate for an adult. In a usual administration, enzalutamide is administered at 160 mg once a day. If necessary, the dose may be increased or decreased. In the present invention, the daily dose of enzalutamide is preferably from 80 to 240 mg, more preferably from 80 to 160 mg, more preferably from 120 to 160 mg, particularly preferably 160 mg.

The recommended dose of darolutamide administered alone is usually 1200 mg/day as abiraterone acetate for an adult. In a usual administration, darolutamide is administered at 600 mg twice a day. If necessary, the dose may be increased or decreased. In the present invention, the daily dose of darolutamide is preferably from 600 to 1800 mg, more preferably from 600 to 1,200 mg, more preferably from 900 to 1200 mg, particularly preferably 1,200 mg.

The recommended dose of apalutamide administered alone is usually 240 mg/day as abiraterone acetate for an

adult. In a usual administration, apalutamide is administered at 240 mg once a day. If necessary, the dose may be increased or decreased. In the present invention, the daily dose of apalutamide is preferably from 120 to 360 mg, more preferably from 120 to 240 mg, more preferably from 180 to 240 mg, particularly preferably 240 mg.

In the present invention, the "recommended dose" is a dose which brings about the largest therapeutic effect in a safely available range determined by clinical trials or the like. Specific examples thereof include doses approved, recommended, or advised by public institutions or organizations such as Pharmaceuticals and Medical Devices Agency (PMDA), Food and Drug Administration (FDA), or European Medicines Agency (EMA) and described in package inserts, interview forms, treatment guidelines, or the like. A dose approved by any of the public institutions PMDA, FDA, and EMA is preferred.

The administration schedules of the compound 1 or the salt thereof and the antiandrogen agent of the present invention may be appropriately selected depending on a cancer type, a disease stage, and the like.

For the compound 1 or the salt thereof, an administration schedule of continuous administration for 5 days and a drug holiday of 2 days which are repeated is preferred. A 4-week administration cycle, involving administration for 5 days followed by a drug holiday of 2 days per week, is defined as one cycle, and an administration schedule with this cycle repeated is most preferred.

For the antiandrogen agent, the recommended administration schedule of each drug is preferred, and daily administration is most preferred as the administration schedules of abiraterone, enzalutamide, darolutamide, and apalutamide.

The order of administration of the compound 1 or the salt thereof and the antiandrogen agent may be appropriately selected depending on a cancer type, a disease stage, and the like. Either of these drugs may be administered first, or these drugs may be administered concurrently. Herein, when these drugs are not administered concurrently, the dosing interval between the drugs may be appropriately selected as long as an enhancing effect on an antitumor effect is exerted. The dosing interval is preferably from 1 to 7 days, more preferably from 1 to 5 days, particularly preferably from 1 to 2 days.

Examples of a suitable combination of the doses per day on an administration day of the compound 1 or the salt thereof and the antiandrogen agent according to the present invention for an adult usually include the following:

50 to 100% of the recommended dose of the compound 1 or the salt thereof administered alone, and 100% of the recommended dose of the antiandrogen agent administered alone;
75 to 100% of the recommended dose of the compound 1 or the salt thereof administered alone, and 100% of the recommended dose of the antiandrogen agent administered alone;
50% of the recommended dose of the compound 1 or the salt thereof administered alone, and 100% of the recommended dose of the antiandrogen agent administered alone;
75% of the recommended dose of the compound 1 or the salt thereof administered alone, and 100% of the recommended dose of the antiandrogen agent administered alone;
100% of the recommended dose of the compound 1 or the salt thereof administered alone, and 100% of the recommended dose of the antiandrogen agent administered alone;
50 to 100% of the recommended dose of the compound 1 or the salt thereof administered alone, and 100% of the recommended dose of abiraterone administered alone;
50 to 100% of the recommended dose of the compound 1 or the salt thereof administered alone, and 100% of the recommended dose of enzalutamide administered alone;
50 to 100% of the recommended dose of the compound 1 or the salt thereof administered alone, and 100% of the recommended dose of darolutamide administered alone;
50 to 100% of the recommended dose of the compound 1 or the salt thereof administered alone, and 100% of the recommended dose of apalutamide administered alone;
75 to 100% of the recommended dose of the compound 1 or the salt thereof administered alone, and 100% of the recommended dose of abiraterone administered alone;
75 to 100% of the recommended dose of the compound 1 or the salt thereof administered alone, and 100% of the recommended dose of enzalutamide administered alone;
75 to 100% of the recommended dose of the compound 1 or the salt thereof administered alone, and 100% of the recommended dose of darolutamide administered alone;
75 to 100% of the recommended dose of the compound 1 or the salt thereof administered alone, and 100% of the recommended dose of apalutamide administered alone;
50% of the recommended dose of the compound 1 or the salt thereof administered alone, and 100% of the recommended dose of abiraterone administered alone;
50% of the recommended dose of the compound 1 or the salt thereof administered alone, and 100% of the recommended dose of enzalutamide administered alone;
50% of the recommended dose of the compound 1 or the salt thereof administered alone, and 100% of the

recommended dose of darolutamide administered alone;
50% of the recommended dose of the compound 1 or the salt thereof administered alone, and 100% of the recommended dose of apalutamide administered alone;
75% of the recommended dose of the compound 1 or the salt thereof administered alone, and 100% of the recommended dose of abiraterone administered alone;
75% of the recommended dose of the compound 1 or the salt thereof administered alone, and 100% of the recommended dose of enzalutamide administered alone;
75% of the recommended dose of the compound 1 or the salt thereof administered alone, and 100% of the recommended dose of darolutamide administered alone;
75% of the recommended dose of the compound 1 or the salt thereof administered alone, and 100% of the recommended dose of apalutamide administered alone;
100% of the recommended dose of the compound 1 or the salt thereof administered alone, and 100% of the recommended dose of abiraterone administered alone;
100% of the recommended dose of the compound 1 or the salt thereof administered alone, and 100% of the recommended dose of enzalutamide administered alone;
100% of the recommended dose of the compound 1 or the salt thereof administered alone, and 100% of the recommended dose of darolutamide administered alone;
100% of the recommended dose of the compound 1 or the salt thereof administered alone, and 100% of the recommended dose of apalutamide administered alone;
80 to 160 mg/day as the compound 1 of the compound 1 or the salt thereof, and 1000 mg/day as abiraterone acetate of abiraterone;
80 to 160 mg/day as the compound 1 of the compound 1 or the salt thereof, and 160 mg/day of enzalutamide;
80 to 160 mg/day as the compound 1 of the compound 1 or the salt thereof, and 1200 mg/day of darolutamide;
80 to 160 mg/day as the compound 1 of the compound 1 or the salt thereof, and 240 mg/day of apalutamide;
120 to 160 mg/day as the compound 1 of the compound 1 or the salt thereof, and 1000 mg/day as abiraterone acetate of abiraterone;
120 to 160 mg/day as the compound 1 of the compound 1 or the salt thereof, and 160 mg/day of enzalutamide;
120 to 160 mg/day as the compound 1 of the compound 1 or the salt thereof, and 1200 mg/day of darolutamide;
120 to 160 mg/day as the compound 1 of the compound 1 or the salt thereof, and 240 mg/day of apalutamide;
80 mg/day as the compound 1 of the compound 1 or the salt thereof, and 1000 mg/day as abiraterone acetate of abiraterone;
80 mg/day as the compound 1 of the compound 1 or the salt thereof, and 160 mg/day of enzalutamide;
80 mg/day as the compound 1 of the compound 1 or the salt thereof, and 1200 mg/day of darolutamide;
80 mg/day as the compound 1 of the compound 1 or the salt thereof, and 240 mg/day of apalutamide;
120 mg/day as the compound 1 of the compound 1 or the salt thereof, and 1000 mg/day as abiraterone acetate of abiraterone;
120 mg/day as the compound 1 of the compound 1 or the salt thereof, and 160 mg/day of enzalutamide;
120 mg/day as the compound 1 of the compound 1 or the salt thereof, and 1200 mg/day of darolutamide;
120 mg/day as the compound 1 of the compound 1 or the salt thereof, and 240 mg/day of apalutamide;
160 mg/day as the compound 1 of the compound 1 or the salt thereof, and 1000 mg/day as abiraterone acetate of abiraterone;
160 mg/day as the compound 1 of the compound 1 or the salt thereof, and 160 mg/day of enzalutamide;
160 mg/day as the compound 1 of the compound 1 or the salt thereof, and 1200 mg/day of darolutamide; and
160 mg/day as the compound 1 of the compound 1 or the salt thereof, and 240 mg/day of apalutamide.

Examples of a suitable 7-day administration schedule according to the present invention for an adult usually include the following, and this 7-day administration schedule is performed once or two or more repeated times:

the compound 1 or the salt thereof is continuously administered at from 80 to 160 mg as the compound 1 once a day for 5 days, followed by a drug holiday of 2 days, and abiraterone is continuously administered at 1000 mg as abiraterone acetate once a day for 7 days;
the compound 1 or the salt thereof is continuously administered at from 80 to 160 mg as the compound 1 once a day for 5 days, followed by a drug holiday of 2 days, and enzalutamide is continuously administered at 160 mg once a day for 7 days;
the compound 1 or the salt thereof is continuously administered at from 80 to 160 mg as the compound 1 once a day for 5 days, followed by a drug holiday of 2 days, and darolutamide is continuously administered at 600 mg twice a day for 7 days;
the compound 1 or the salt thereof is continuously administered at from 80 to 160 mg as the compound 1 once a day for

5 days, followed by a drug holiday of 2 days, and apalutamide is continuously administered at 240 mg once a day for 7 days;
the compound 1 or the salt thereof is continuously administered at from 120 to 160 mg as the compound 1 once a day for 5 days, followed by a drug holiday of 2 days, and abiraterone is continuously administered at 1000 mg as abiraterone acetate once a day for 7 days;
the compound 1 or the salt thereof is continuously administered at from 120 to 160 mg as the compound 1 once a day for 5 days, followed by a drug holiday of 2 days, and enzalutamide is continuously administered at 160 mg once a day for 7 days;
the compound 1 or the salt thereof is continuously administered at from 120 to 160 mg as the compound 1 once a day for 5 days, followed by a drug holiday of 2 days, and darolutamide is continuously administered at 600 mg twice a day for 7 days;
the compound 1 or the salt thereof is continuously administered at from 120 to 160 mg as the compound 1 once a day for 5 days, followed by a drug holiday of 2 days, and apalutamide is continuously administered at 240 mg once a day for 7 days;
the compound 1 or the salt thereof is continuously administered at 80 mg as the compound 1 once a day for 5 days, followed by a drug holiday of 2 days, and abiraterone is continuously administered at 1000 mg as abiraterone acetate once a day for 7 days;
the compound 1 or the salt thereof is continuously administered at 80 mg as the compound 1 once a day for 5 days, followed by a drug holiday of 2 days, and enzalutamide is continuously administered at 160 mg once a day for 7 days;
the compound 1 or the salt thereof is continuously administered at 80 mg as the compound 1 once a day for 5 days, followed by a drug holiday of 2 days, and darolutamide is continuously administered at 600 mg twice a day for 7 days;
the compound 1 or the salt thereof is continuously administered at 80 mg as the compound 1 once a day for 5 days, followed by a drug holiday of 2 days, and apalutamide is continuously administered at 240 mg once a day for 7 days;
the compound 1 or the salt thereof is continuously administered at 120 mg as the compound 1 once a day for 5 days, followed by a drug holiday of 2 days, and abiraterone is continuously administered at 1000 mg as abiraterone acetate once a day for 7 days;
the compound 1 or the salt thereof is continuously administered at 120 mg as the compound 1 once a day for 5 days, followed by a drug holiday of 2 days, and enzalutamide is continuously administered at 160 mg once a day for 7 days;
the compound 1 or the salt thereof is continuously administered at 120 mg as the compound 1 once a day for 5 days, followed by a drug holiday of 2 days, and darolutamide is continuously administered at 600 mg twice a day for 7 days;
the compound 1 or the salt thereof is continuously administered at 120 mg as the compound 1 once a day for 5 days, followed by a drug holiday of 2 days, and apalutamide is continuously administered at 240 mg once a day for 7 days;
the compound 1 or the salt thereof is continuously administered at 160 mg as the compound 1 once a day for 5 days, followed by a drug holiday of 2 days, and abiraterone is continuously administered at 1000 mg as abiraterone acetate once a day for 7 days;
the compound 1 or the salt thereof is continuously administered at 160 mg as the compound 1 once a day for 5 days, followed by a drug holiday of 2 days, and enzalutamide is continuously administered at 160 mg once for 7 days;
the compound 1 or the salt thereof is continuously administered at 160 mg as the compound 1 once a day for 5 days, followed by a drug holiday of 2 days, and darolutamide is continuously administered at 600 mg twice a day for 7 days; and
the compound 1 or the salt thereof is continuously administered at 160 mg as the compound 1 once a day for 5 days, followed by a drug holiday of 2 days, and apalutamide is continuously administered at 240 mg once a day for 7 days.

The administration of the compound 1 or the salt thereof and the antiandrogen agent in combination brings about an excellent therapeutic effect as compared with each drug administered alone. Examples of items which may be evaluated for the therapeutic effect of the present invention include, but are not particularly limited to, the following: radiographic progression-free survival (rPFS), objective response rate (ORR), disease control rate (DCR), duration of response (DoR), prostate-specific antigen (PSA) response rate (PSA30 and PSA50) and time to progression, time to symptomatic skeletal-related event (SSRE), change in bone scan index (BSI), score fluctuation of brief pain inventory-short form (BPI-SF), fluctuation of bone metabolism markers, time to next treatment (TTNT) for prostate cancer, time to initiation of neoadjuvant chemotherapy (TTNC) for prostate cancer, time to the second progression (PFS2), overall survival (OS), and best overall response (BOR) based on RECIST (which may include complete response (CR), partial response (PR), and stable disease (SD)).

In the present invention, the compound 1 or the salt thereof and the antiandrogen agent may be formulated in two or more dosage forms into which the individual active ingredients are divided, or may be formulated together in one dosage form (i.e., formulated as a combination drug), on the basis of the respective administration forms or administration schedules of the active ingredients. The individual preparations may be produced and sold together in one package suitable for combined use, or the individual preparations may be produced and sold in separate packages.

The administration form of the antitumor agent or the like of the present invention is not particularly limited and can be appropriately selected depending on a therapeutic purpose. Specific examples thereof may include oral formulations (tablets, coated tablets, powders, granules, capsules, solutions, and the like), injections, suppositories, patches, and ointments. An oral formulation is preferred.

Such preparations in various dosage forms can usually be prepared by a known method using, if necessary, a pharmaceutically acceptable carrier. Examples of such a carrier may include various carriers widely used in usual drugs, for example, excipients, binders, disintegrants, lubricants, diluents, solubilizers, suspending agents, tonicity agents, pH adjusters, buffers, stabilizers, colorants, flavors, and odor-masking agents.

The present invention also relates to an antitumor effect enhancer for an antiandrogen agent, comprising compound 1 or a salt thereof as an active ingredient. Herein, the "enhancement of an antitumor effect of an antiandrogen agent" by the compound 1 or the salt thereof" means that combined use of the compound 1 or the salt thereof and the antiandrogen agent exerts an antitumor effect greater than that of the single antiandrogen agent. The antitumor effect enhancer has the dosage form of the antitumor agent described above.

The present invention also relates to an antitumor agent comprising compound 1 or a salt thereof, in which the antitumor agent is administered in combination with an antiandrogen agent. The antitumor agent has the dosage form described above.

The present invention also relates to an antitumor agent comprising a combination of compound 1 or a salt thereof and an antiandrogen agent. The antitumor agent has the dosage form described above.

The present invention also relates to a kit preparation comprising an instruction manual stating that compound 1 or a salt thereof and an antiandrogen agent are administered in combination.

Herein, the "instruction manual" preferably may not be limited as long as it states the dose described above. The instruction manual preferably recommends the dose, regardless of the presence or absence of legal binding power. Specific examples thereof include attached documents and pamphlets. The kit preparation comprising the instruction manual may be one in which the instruction manual is printed or attached to a package, or one in which the instruction manual is enclosed in a package together with the antitumor agent.

## Examples

Hereinafter, the present invention will be described in more detail with reference to Examples. However, the present invention is not limited by these Examples by any means. A person of ordinary skill in the art is capable of making many changes or modifications without departing from the technical idea of the present invention.

### Example 1: *In vitro* combination analysis of compound 1 and abiraterone

Human prostate cancer cells VCaP (American Type Culture Collection) were allowed to proliferate in RPMI-1640 medium (FUJIFILM Wako Pure Chemical Corp.) containing 10% fetal bovine serum (FBS). The cells were maintained at 37°C under 5% $CO_2$ and passaged every 4 to 10 days at a ratio of from 1:2 to 1:5. The number of live cells was measured using CellTiter-Glo2.0(R) Reagent (Promega Corp.).

The cells were recovered by a routine method, suspended in a medium, and seeded in 384-well plates. The number of cells to be seeded was 900 cells/40 μL per well. After incubation overnight at 37°C under 5% $CO_2$, abiraterone and the compound 1 dissolved in DMSO were each added thereto using D300e digital dispenser (Tecan Trading AG). The concentrations of abiraterone were nine concentrations, 0, 0.01, 0.03, 0.1, 0.3, 1, 3, 10, and 30 μM, and the concentrations of the compound 1 were six concentrations, 0, 0.1, 0.3, 1, 3, and 10 μM. All of 54 combinations in total thereof were studied. Four wells were assigned to each combination. In addition, the number of cells at the start of drug treatment (Day 0) was determined by adding 40 μL of CellTiter-Glo2.0(TM) Reagent per well, incubating the cells at room temperature for 10 minutes, and then measuring chemiluminescence using a plate reader, Spark10M (Tecan Trading AG). The cells supplemented with the compounds were further incubated at 37°C for 5 days under 5% $CO_2$, and the number of live cells was measured in the same manner as in Day 0. Cells supplemented with only the evaluation medium (concentrations of both the drugs: 0 μM) were cultured as a control, and the number of live cells was measured (control group).

From the number of live cells, the rate of increase or decrease in the number of cells after the start of drug treatment at each drug treatment concentration was calculated as follows.

(1) Concentration at which the number of cells after drug treatment (Day 5) was larger than that at the start of drug treatment (Day 0), indicating suppression of proliferation

The rate of suppression of cell proliferation compared to the control group of Day 5 was calculated.

Rate of suppression of cell proliferation (%) = (The number of live cells in the test group - The number of live cells in the control group) / (The number of live cells in the control group) × 100

(2) Concentration at which the number of cells after drug treatment (Day 5) was smaller than that at the start of drug treatment (Day 0), indicating cell death

The rate of decrease in the number of cells on Day 5 compared to the number of cells at the start of drug treatment (Day 0) was calculated.

Rate of cell proliferation (%) = (The number of live cells in the test group - The number of live cells at the start of drug treatment) / (The number of live cells in the control group - The number of live cells at the start of drug treatment) × 100

Exemplary results are illustrated in Figure 1.

As a result of analyzing the obtained rate of suppression of cell proliferation (%) by Aspin-Welch t-test, the compound 1 + abiraterone combination treatment group had a significantly higher suppression rate of cell proliferation than the compound 1 alone treatment group, the abiraterone alone treatment group, or the control group, indicating a stronger suppressive effect on cell proliferation.

Example 2: *In vitro* combination analysis of compound 1 and enzalutamide

Human prostate cancer cells VCaP in RPMI-1640 medium containing 10% FBS and human prostate cancer cells 22Rv1 (European Collection of Cell Cultures) in phenol red-free RPMI-1640 medium (Thermo Fisher Scientific Inc.) containing 10% FBS were allowed to proliferate. The cells were maintained at 37°C under 5% $CO_2$ and passaged every 4 to 10 days at a ratio of from 1:2 to 1:5. The cells were recovered by a routine method, suspended in phenol red-free RPMI-1640 containing 10% charcoal-treated fetal bovine serum (hereinafter, referred to as DCC-FBS), and seeded in 384-well plates. The numbers of cells to be seeded were 400 cells/40 μL for 22Rv1 and 900 cells/40 μL for VCaP per well. After incubation overnight at 37°C under 5% $CO_2$, enzalutamide and the compound 1 dissolved in DMSO and dihydrotestosterone (DHT) (final concentration: 0.1 nmol/L) were each added thereto using D300e digital dispenser (Tecan Trading AG), and analyzed in the same manner as in abiraterone.

Exemplary results are illustrated in Figure 2.

As a result of analyzing the obtained rate of suppression of cell proliferation (%) by Aspin-Welch t-test, the compound 1 + enzalutamide combination treatment group had a significantly higher suppression rate of cell proliferation than the compound 1 alone treatment group, the enzalutamide alone treatment group, and the control group, indicating a stronger suppressive effect on cell proliferation.

Example 3: *In vitro* combination analysis of compound 1 and darolutamide

Human prostate cancer cells VCaP in RPMI-1640 medium containing 10% FBS, human prostate cancer cells LNCaP (Kyoto Biken Laboratories, Inc.) in RPMI-1640 medium containing 5% FBS, and 22Rv1 in phenol red-free RPMI-1640 medium containing 10% FBS were allowed to proliferate. The cells were maintained at 37°C under 5% $CO_2$ and passaged every 4 to 10 days at a ratio of from 1:2 to 1:5. The cells were recovered by a routine method, suspended in phenol red-free RPMI-1640 containing 5% (for LNCaP) or 10% (for 22Rv1 and VCaP) DCC-FBS, and seeded in 384-well plates. The numbers of cells to be seeded were 400 cells/40 μL for LNCaP and 22Rv1 and 900 cells/40 μL for VCaP per well. After incubation overnight at 37°C under 5% $CO_2$, darolutamide and the compound 1 dissolved in DMSO and DHT (final concentration: 0.1 nmol/L) were each added thereto using D300e digital dispenser (Tecan Trading AG), and analyzed in the same manner as in abiraterone.

Exemplary results are illustrated in Figures 3-1 and 3-2.

As a result of analyzing the obtained rate of suppression of cell proliferation (%) by Aspin-Welch t-test, the compound 1 + darolutamide combination treatment group had a significantly higher suppression rate of cell proliferation than the compound 1 alone treatment group, darolutamide alone treatment group, and the control group, indicating a stronger suppressive effect on cell proliferation.

Example 4: Evaluation of antitumor effect by combination of compound 1 and enzalutamide in subcutaneous transplanted castration resistant prostate cancer LNCaP model

On the basis of the report of the article (Clin Cancer Res, 2001 7: 2941-8), castration resistant prostate cancer

LNCaP-Xeno-IL-6 cells (reported as LNCaP-IL-6+ cells in the article) were established from human prostate cancer cells LNCaP and used in a test. The LNCaP-Xeno-IL-6 cells were cultured in RPMI-1640 medium containing 5% FBS and human recombinant IL-6 (final concentration: 5 ng/mL). Normal human lung fibroblasts (NHLF) as feeder cells were cultured in FBM medium (Lonza Group AG). The LNCaP-Xeno-IL-6 cells and the NHLF were passaged every 4 to 10 days at a ratio of from 1:3 to 1:5 in a 5% $CO_2$ incubator of 37°C.

A cell suspension containing the LNCaP-Xeno-IL-6 cells at $2 \times 10^6$ cells/0.1 mL and the NHLF at $1 \times 10^6$ cells/0.1 mL was prepared with a 50% Matrigel (Corning Inc.) solution containing DHT (final concentration: 10 nmol/L), and subcutaneously transplanted to near the right posterior rib of six-week-old C.B-17 SCID mice (The Jackson Laboratory Japan, Inc.). Tumor after the transplantation was allowed to grow until a tumor volume (TV) reached 100 $mm^3$. Then, castration treatment was performed.

Digimatic calipers were used in tumor size measurement. The major axis and minor axis of the tumor were measured, and TV was calculated in accordance with the following equation.

$$TV\ (mm^3) = \text{Major axis (mm)} \times \text{Minor axis (mm)} \times \text{Minor axis (mm)} / 2$$

Three days later from the castration operation, six animals were assigned to each group by the stratified randomization method with TV as an index. The day on which grouping (n = 6) was carried out was regarded as Day 1.

The rate of relative change in tumor volume (T/C) was calculated from TV in accordance with the following equation.

T/C (%) = (Average TV of each drug administration group) / (Average TV of a control group) × 100

An electronic balance for an animal was used in body weight measurement. The rate of change in body weight on Day n (BWCn) was calculated from the body weight of Day n (BWn) in accordance with the following equation.

$$\text{Rate of change in body weight, BWCn, (\%)} = (BWn - BW1) / BW1 \times 100$$

Japanese Pharmacopoeia water for injection was added in an appropriate amount such that a hypromellose concentration was 0.5 w/v%. Then, the mixture was stirred using a stirrer for complete dissolution to prepare a 0.5% aqueous hypromellose solution. The compound 1 was ground using Rotation/revolution Mixer (Thinky Corp.), suspended at a predetermined concentration in the 0.5% aqueous hypromellose solution, and ultrasonically treated to prepare a uniform suspension. This suspension was orally administered at 10 mg/kg/day or 14 mg/kg/day as the compound 1 once a day for 5 consecutive days, followed by a drug holiday of 2 days. This step was repeated three times.

Enzalutamide was ground using Rotation/revolution Mixer, suspended at a predetermined concentration in the 0.5% aqueous hypromellose solution, and ultrasonically treated to prepare a uniform suspension. This suspension was orally administered at 10 mg/kg/day as enzalutamide once a day for 21 consecutive days.

DMSO was added to NVP-AUY922 to obtain a concentration of 71.4 mg/mL. Then, a homogeneous solution was prepared by ultrasonic treatment. This solution was diluted 20-fold using a Tween 20/physiological saline mixed solution of Tween 20 and physiological saline mixed at a ratio of 1:18. This solution was intravenously administered at 35.7 mg/kg/day once a day and every other day three times, followed by a drug holiday of 1 day. This step was repeated three times.

Japanese Pharmacopoeia water for injection was added in an appropriate amount such that a hydroxypropyl-β-cyclodextrin (HP-β-CD) concentration was 17.5 w/v%. Then, the mixture was stirred using a stirrer for complete dissolution to prepare a 17.5% aqueous HP-β-CD solution. The 17.5% aqueous HP-β-CD solution was added to AT13387 monolactate to obtain a concentration of 9 mg/mL as AT13387. A homogeneous solution was prepared by ultrasonic treatment. This solution was intraperitoneally administered at 90 mg/kg/day once a day and once a week. This step was repeated three times.

The results are shown in Figure 4 and Table 1.

[Table 1]

| Group | Dose (mg/kg/day) | Dosing schedule | Route | Tumor volume ($mm^3$) Day 22 | Dunnett | Aspin-Welch | | | | |
|---|---|---|---|---|---|---|---|---|---|---|
| | | | | Mean ± SE | vs Control | vs Enz | vs Comp1 | vs AUY | vs AT | T/C ratio (%) |
| Control | 0 | Day 1-21 | p.o. | 723.9 ± 81.4 | NA | - | - | - | - | 100 |
| Enzalutamide (ENZ) | 10 | Day 1-21 | p.o. | 265.8 ± 32.4 | 0.000 | NA | - | - | - | 37 |
| Compound 1 (Comp1) | 10 | Day 1-5,8-12,15-19 | p.o. | 532.9 ± 78.4 | 0.229 | - | NA | - | - | 74 |
| Comp1 | 14 | Day 1-5,8-12,15-19 | p.o. | 542.8 ± 36.6 | 0.421 | - | NA | - | - | 75 |
| NVP-AUY922 (AUY) | 35.7 | Day 1,3,5,10,12,15,17,19 | i.v. | 563.3 ± 81.0 | 0.382 | - | - | NA | - | 78 |
| AT13387 (AT) | 90 | Day 1,8,15 | i.p. | 438.9 ± 58.0 | 0.011 | - | - | - | NA | 61 |
| ENZ + Comp1 | 10+10 | Day 1-21+ Day 1-5,8-12,15-19 | p.o.+p.o. | 181.7 ± 7.0 | 0.000 | 0.046 | 0.000 | - | - | 25 |
| ENZ + Comp1 | 10+14 | Day 1-21+ Day 1-5,8-12,15-19 | p.o.+p.o. | 158.6 ± 7.5 | 0.000 | 0.012 | 0.000 | - | - | 22 |
| ENZ +AUY | 10+35.7 | Day 1-21+Day 1,3,5,8,10,12,15,17 | p.o.+i.v. | 192.4 ± 21.1 | 0.000 | 0.094 | - | 0.000 | - | 27 |
| ENZ +AT | 10+90 | Day 1-21+Day 1,8,15 | p.o.+i.p. | 190.1 ± 13.8 | 0.000 | 0.074 | - | - | 0.002 | 26 |

As a result of analyzing TV of Day 22 in each group by Dunnett's test, the single-drug group of the AT13387 group or the enzalutamide group, and the compound 1 + enzalutamide, NVP-AUY922 + enzalutamide, or AT13387 + enzalutamide combination administration group had significantly low TV as compared with the control group, indicating an antitumor effect. The single-drug groups of the compound 1 group and the NVP-AUY922 group were confirmed to have no significant difference from the control group, though these groups tended to have low TV.

As a result of further analysis by Aspin-Welch t-test, the compound 1 + enzalutamide combination administration group had significantly low TV as compared with the single-drug group of the compound 1 group or the enzalutamide group, indicating a stronger antitumor effect. The average rate of change in body weight in the combination administration group involved no enhancement of toxicity as compared with the single-drug group of the compound 1 group or the enzalutamide group.

On the other hand, the NVP-AUY922 + enzalutamide or AT13387 + enzalutamide combination administration group was confirmed to have no significant difference from the single-drug group of the enzalutamide group.

Example 5: Evaluation of antitumor effect by combination of compound 1 and enzalutamide in subcutaneous transplanted castration resistant prostate cancer VCaP model

Human prostate cancer cells VCaP were cultured in RPMI-1640 medium containing 10% FBS. The cells were passaged every 4 to 10 days at a ratio of from 1:2 to 1:5 in a 5% CO2 incubator of 37°C.

A cell suspension of the VCaP cells at $5 \times 10^6$ cells/0.1 mL was prepared with a 50% Matrigel (Corning Inc.) solution and subcutaneously transplanted to near the right posterior rib of seven-week-old C.B-17 SCID mice (The Jackson Laboratory Japan, Inc.).

Tumor after the transplantation was allowed to grow until a tumor volume (TV) reached 200 $mm^3$. Then, castration treatment was performed. Tumor after the castration was allowed to grow until a tumor volume (TV) reached 220 to 390 $mm^3$ (2 to 3 weeks). Six animals were assigned to each group by the stratified randomization method with TV as an index. The day on which grouping (n = 6) was carried out was regarded as Day 0. Tumor size measurement, body weight measurement, and the preparation of a suspension of the compound 1 or enzalutamide were the same as in Example 4.

The results are shown in Figure 5 and Table 2.

[Table 2]

| Group | Dose (mg/kg/day) | Dosing schedule | Route | Tumor volume ($mm^3$) Day 29 | Dunnett | Aspin-Welch | | T/C (%) |
|---|---|---|---|---|---|---|---|---|
| | | | | Mean ± SE | vs Control | vs Comp. 1 | vs Enz | |
| Control | - | Day 1-28 | p.o. | 780.9 ± 56.9 | NA | v | - | 100 |
| Enzalutamide | 10 | Day 1-28 | p.o. | 783.8 ± 96.1 | 1.000 | - | NA | 100 |
| Compound 1 | 10 | Day 1-5,8-12,15-19,22-26 | p.o. | 681.8 ± 63.6 | 0.763 | NA | - | 87 |
| Compound 1 | 14 | Day 1-5,8-12,15-19,22-26 | p.o. | 690.9 ± 96.5 | 0.672 | NA | - | 88 |
| Enzalutamide + Compound 1 | 10 + 10 | Day 1-28 + Day 1-5,8-12,15-19,22-26 | p.o. + p.o. | 406.3 ± 22.9 | 0.000 | 0.001 | 0.003 | 52 |
| Enzalutamide + Compound 1 | 10 + 14 | Day 1-28 + Day 1-5,8-12,15-19,22-26 | p.o. + p.o. | 428.3 ± 28.3 | 0.001 | 0.033 | 0.004 | 55 |

As a result of analyzing TV of Day 29 in each group by Dunnett's test, the compound 1 + enzalutamide combination administration group had significantly low TV as compared with the control group, indicating an antitumor effect. On the other hand, the compound 1 group was confirmed to have no significant difference from the control group, though this group tended to have low TV. The single-drug group of the enzalutamide group exhibited no antitumor effect.

As a result of further analysis by Aspin-Welch t-test, the compound 1 + enzalutamide combination administration group had significantly low TV as compared with the single-drug group of the compound 1 group or the enzalutamide

group, indicating a stronger antitumor effect. The average rate of change in body weight in the combination administration group involved no enhancement of toxicity as compared with the single-drug group of the compound 1 group or the enzalutamide group.

Example 6: Evaluation of antitumor effect by combination of compound 1 and darolutamide in subcutaneous transplanted castration resistant prostate cancer LNCaP model

The same models as in Example 4 were used. However, five animals were assigned to each group in this Example. The day on which grouping (n = 5) was carried out was regarded as Day 0. Tumor size measurement, body weight measurement, and the preparation of a suspension of the compound 1 were the same as in Example 4.

Japanese Pharmacopoeia water for injection was added in an appropriate amount such that a hypromellose concentration was 0.5 w/v%. Then, the mixture was stirred using a stirrer for complete dissolution to prepare a 0.5% aqueous hypromellose solution.

Darolutamide was ground using Rotation/revolution Mixer, suspended at a predetermined concentration in the 0.5% aqueous hypromellose solution, and ultrasonically treated to prepare a uniform suspension. This suspension was orally administered at 50 mg/kg/day as darolutamide twice a day for 21 consecutive days.

The results are shown in Figure 6 and Table 3.

[Table 3]

| Group | Dose (mg/kg/day) | Dosing schedule | Route | Tumor volume ($mm^3$) Day 22 | Dunnett | Aspin-Welch | | T/C (%) |
|---|---|---|---|---|---|---|---|---|
| | | | | Mean ± SE | vs Control | vs DAR | vs Comp1 | |
| Control | - | Day 1-21 | p.o. | 372.9 ± 71.9 | NA | - | - | 100 |
| Darolutamide (DAR) | 50 | Day 1-21 | p.o. | 177.2 ± 22.5 | 0.0357 | NA | - | 48 |
| Compound 1 (Comp1) | 10 | Day 1-5,8-12,15-19 | p.o. | 217.3 ± 41.4 | 0.1233 | - | NA | 58 |
| Comp1 | 14 | Day 1-5,8-12,15-19 | p.o. | 216.8 ± 20.9 | 0.2147 | - | NA | 58 |
| DAR + Comp1 | 50+10 | Day 1-21 + Day 1-5,8-12,15-19 | p.o. + p.o. | 94.1 ± 8.0 | 0.0001 | 0.0042 | 0.0255 | 25 |
| DAR + Comp1 | 50+14 | Day 1-21 + Day 1-5,8-12,15-19 | p.o. + p.o. | 120.4 ± 23.7 | 0.0004 | 0.1105 | 0.0287 | 32 |

As a result of analyzing TV of Day 22 in each group by Dunnett's test, the single-drug group of the darolutamide group, and the compound 1 + darolutamide combination administration group had significantly low TV as compared with the control group, indicating an antitumor effect. The single-drug group of the compound 1 was confirmed to have no significant difference from the control group, though this group tended to have low TV.

As a result of further analysis by Aspin-Welch t-test, the compound 1 + darolutamide combination administration group had significantly low TV as compared with the single-drug group of the compound 1 group, indicating a stronger antitumor effect. The compound 1 (10 mg/kg/day) + darolutamide combination administration group had significantly low TV as compared with the single-drug group of the darolutamide group, indicating a stronger antitumor effect. The average rate of change in body weight in the combination administration group involved no enhancement of toxicity as compared with the single-drug group of the compound 1 group or the darolutamide group.

Example 7: Evaluation of antitumor effect by combination of compound 1 and abiraterone in subcutaneously castration resistant prostate cancer VCaP-transplanted model

The same models as in Example 5 were used. However, the day on which grouping (n = 6) was carried out was regarded as Day 1. Tumor size measurement, body weight measurement, and the preparation of a suspension of the

compound 1 and a solution of AT13387 were the same as in Example 4.

Japanese Pharmacopoeia water for injection was added in an appropriate amount such that a hypromellose concentration was 0.5 w/v%. Then, the mixture was stirred using a stirrer for complete dissolution to prepare a 0.5% aqueous hypromellose solution.

Abiraterone acetate was ground using Rotation/revolution Mixer, suspended at a predetermined concentration in the 0.5% aqueous hypromellose solution, and ultrasonically treated to prepare a uniform suspension. This suspension was orally administered at 200 mg/kg/day as abiraterone once a day for 21 consecutive days.

The results are shown in Figure 7 and Table 4.

[Table 4]

| Group | Dose (mg/kg/day) | Dosing schedule | Route | Tumor volume ($mm^3$) Day 29 | Dunnett | Aspin-Welch | | | T/C (%) |
|---|---|---|---|---|---|---|---|---|---|
| | | | | Mean ± SE | vs Control | vs ABI | vs Comp1 | vs AT | |
| Control | - | Day 1-28 | p.o. | 486.6 ± 62.9 | NA | - | - | - | 100 |
| Abiraterone (ABI) | 200 | Day 1-28 | p.o. | 345.7 ± 18.3 | 0.0408 | NA | - | - | 71 |
| Compound 1 (Comp1) | 10 | Day 1-5,8-12,15-19,22-26 | p.o. | 355.9 ± 13.5 | 0.0823 | - | NA | - | 73 |
| Comp1 | 14 | Day 1-5,8-12,15-19,22-26 | p.o. | 350.7 ± 26.1 | 0.0459 | - | NA | - | 72 |
| AT13387 (AT) | 45 | Day 1,2,8,9,15,16,22,23 | i.p. | 288.0 ± 23.5 | 0.0002 | - | - | NA | 59 |
| AT | 90 | Day 1,8,15,22 | i.p. | 270.2 ± 22.7 | 0.0000 | - | - | NA | 56 |
| ABI+ Comp1 | 200 + 10 | Day 1-28 + Day 1-5,8-12,15-19,22-26 | p.o. + p.o. | 247.3 ± 15.6 | 0.0000 | 0.0016 | 0.0006 | NA | 51 |
| ABI + Comp1 | 200 + 14 | Day 1-28 + Day 1-5,8-12,15-19,22-26 | p.o. + p.o. | 189.0 ± 10.0 | 0.0000 | 0.0000 | 0.0002 | NA | 39 |
| ABI+ AT | 200 + 45 | Day 1-28 + Day 1,2,8,9,15,16,22,23 | p.o. + i.p. | 252.1 ± 22.4 | 0.0000 | 0.0158 | NA | 0.3417 | 52 |
| ABI + AT | 200 + 90 | Day 1-28 + Day 1,8,15,22 | p.o. + i.p. | 252.2 ± 16.3 | 0.0000 | 0.0039 | NA | 0.5957 | 52 |

As a result of analyzing TV of Day 29 in each group by Dunnett's test, the single-drug group of the AT13387 group, the compound 1 14 mg/kg/day group, or the abiraterone group, and the compound 1 + abiraterone or AT13387 + abiraterone combination administration group had significantly low TV as compared with the control group, indicating an antitumor effect. The single-drug group of the compound 1 10 mg/kg/day group was confirmed to have no significant difference from the control group, though this group tended to have low TV.

As a result of further analysis by Aspin-Welch t-test, the compound 1 + abiraterone combination administration group had significantly low TV as compared with the single-drug group of the compound 1 group or the abiraterone group, indicating a stronger antitumor effect. The average rate of change in body weight in the combination administration group involved no enhancement of toxicity as compared with the single-drug group of the compound 1 group or the abiraterone group.

On the other hand, the AT13387 + abiraterone combination administration group was confirmed to have no significant difference from the single-drug group of the AT13387 group, though this group had significantly low TV as compared with the single-drug group of the abiraterone group, indicating a stronger antitumor effect.

Example 8: Execution of Phase 2 clinical trial to evaluate combination therapy using pimitespib (compound 1) and enzalutamide targeting CRPC patient

[Table 5-1]

| Purpose and evaluation item: | |
|---|---|
| Purpose | Evaluation item |
| Primary | |
| **Feasibility part**<br>· Evaluate the tolerability and safety of combination therapy with pimitespib and enzalutamide and determine a maximum tolerated dose (MTD) | · Dose limiting toxicity (DLT) during the period of cycle 1 |
| **Expansion part**<br>· Evaluate the effectiveness of combination therapy with pimitespib and enzalutamide | Radiographic progression-free survival (rPFS) based on RECIST v1.1 and PCWG3 by the independent radiological review |
| Secondary | |
| **Feasibility part**<br>· Evaluate the pharmacokinetics (PK) of combination therapy with pimitespib and enzalutamide | · PK parameters (pimitespib, its metabolite, enzalutamide, and its metabolite) |
| **Feasibility part and Expansion part**<br>· Evaluate the safety of combination therapy with pimitespib and enzalutamide | · Adverse event<br>· Adverse drug reaction<br>· Laboratory abnormalities<br>· Vital sign<br>· 12-lead electrocardiogram |

[Table 5-2]

| | |
|---|---|
| **Feasibility part and Expansion part**<br>· Evaluate the effectiveness of combination therapy with pimitespib and enzalutamide | · rPFS, objective response rate (ORR), disease control rate (DCR), and duration of response (DoR) based on RECIST v1.1 and PCWG3 by the independent radiological review (Feasibility part)<br>· rPFS, ORR, DCR, and DoR based on RECIST v1.1 and PCWG3 by the physician-in-charge<br>· Prostate-specific antigen (PSA) response rate (PSA30 and PSA50) and time to progression<br>· time to symptomatic skeletal-related event (SSRE)<br>· Change in bone scan index (BSI)<br>· Score fluctuation of brief pain inventory-short form (BPI-SF)<br>· Fluctuation of bone metabolism markers<br>· Time to next treatment (TTNT) for prostate cancer and time to initiation of neoadjuvant chemotherapy (TTNC) for prostate cancer<br>· Time from enrollment to the second progression (PFS2)<br>· Overall survival (OS) |

Method:

A clinical trial using pimitespib and enzalutamide in combination is conducted targeting metastatic castration resistant prostate cancer patients having a treatment history of one regimen of a novel hormone agent. This test is composed of two parts.

· Feasibility part: A primary purpose is to evaluate the tolerability and safety of combination therapy with pimitespib and determine a maximum tolerated dose (MTD). This part is carried out in nonrandomized design.

· Expansion part: A primary purpose is to evaluate the effectiveness of combination therapy with pimitespib (radiographic progression-free survival (rPFS) based on RECIST v1.1 and PCWG3 by the independent radiological review). This part is carried out in randomized design. Enrollment is started after the completion of dose limiting toxicity (DLT) evaluation in the feasibility part.

Target sample size (anticipated):

In the feasibility part, 12 individuals at maximum are evaluated as DLT-evaluable cases. In the expansion part, 20 individuals (10 individuals per group) are enrolled as populations to be evaluated for effectiveness.

Diagnosis and key inclusion criteria:

· Being a metastatic CRPC patient aged 18 or over.
· having a treatment history with one type of novel hormone agent (abiraterone for the feasibility part and a novel hormone agent containing abiraterone for the expansion part) as treatment for metastatic or nonmetastatic CRPC or metastatic castration-sensitive prostate cancer.
· Naive to chemotherapy (cytotoxic anticancer drugs, and the like) and Ra-223 administration as treatment for metastatic CRPC, had treatment with a novel hormone agent as the most recent previous medication, and documented disease progression during the treatment. Herein, the "disease progression" includes, for example, 1. progression of PSA: PSA value increased in two or more consecutive measurements taken at intervals of one week or more, and the increase in PSA value was 1 ng/mL or more (based on PCWG3); 2. progression of soft tissue lesions: determined to be progression of disease in accordance with the definition of RECIST v1.1; and 3. progression of bone lesions: two or more new lesions appeared on bone scintigraphy (based on PCWG3).

Dose and administration method:

· Feasibility part: Pimitespib is orally administered at a dose of 160 mg (level 1) or 120 mg (level -1) once a day on an empty stomach (except for at least 1 hour before or 2 hours after a meal) in a schedule of administration for 5 consecutive days followed by a drug holiday of 2 days. Enzalutamide is orally administered at 160 mg once a day.
· Expansion part: Pimitespib is orally administered at a dose not exceeding MTD determined in the feasibility part once a day on an empty stomach (except for at least 1 hour before or 2 hours after a meal) in a schedule of administration for 5 consecutive days followed by a drug holiday of 2 days. Enzalutamide is orally administered at 160 mg once a day.
· Pimitespib and enzalutamide can be taken at the same time.

Treatment period:

One cycle involves 28 days, and the test treatment continues until applying to discontinuation criteria.

Evaluation criteria:

Effectiveness: RECIST Version 1.1 and PCWG3

Safety: CTCAE Version 5.0

Statistical approach:

Populations to be analyzed in this test are as follows.

[Table 6]

| Population to be analyzed | Definition |
|---|---|
| Enrolled case | All enrolled patients |
| All-treated population (ATP) | Among the enrolled patients, all patients given either pimitespib or enzalutamide one or more times |
| DLT-evaluable case | All ATP patients of the feasibility part except for:<br>· Patients given prohibited medication before manifestation of DLT in cycle 1.<br>· Patients confirmed to have no DLT in cycle 1 and having less than 75% of the prescribed number of pimitespib administration days for reasons other than test-related adverse event (TRAE).<br>Patients whom enzalutamide could not be administered for 21 days. Skipped administration ascribable to TRAE or other procedures for administration are regarded as having received the administration.<br>· Patients who did not receive any examination or observation prescribed in cycle 1, confirmed to be necessary for DLT evaluation.<br>· Patients who withdrew the consent to participate in the test or discontinued administration of the test treatment, for reasons other than TRAE in cycle 1. |
| Population to be analyzed for effectiveness | All ATP patients who received one or more evaluations of effectiveness evaluation items (either primary evaluation items or secondary evaluation items) after study drug administration |
| Population to be analyzed for PK | Among ATP patients of the feasibility part, all patients evaluable for the plasma concentration of any of pimitespib, its metabolite, enzalutamide, and its metabolite. |
| Population to be analyzed for biomarker | In the population to be analyzed for effectiveness, all patients from which proper data for biomarker analysis was obtained. |

Analysis of primary evaluation item

- Feasibility part
  The number of DLT cases of cycle 1, the incidence rate, and the 95% confidence interval (CI) of the incidence rate are calculated at each dose level targeting DLT-evaluable cases. Every DLT is listed on a patient basis.

- Expansion part

Effectiveness is analyzed targeting the population to be evaluated for effectiveness.

The hazard ratio and 95% CI of rPFS by the independent radiological review are estimated for the pimitespib + enzalutamide combination therapy group against the enzalutamide monotherapy group using the Cox proportional hazard model.

A two-sided p value for the comparison of rPFS by the independent radiological review is calculated by use of the log rank test.

An rPFS curve by the independent radiological review is shown by use of the Kaplan-Meier method. A median value and 95% CI as well as restricted mean survival time and 95% CI are estimated. The number of populations at risk and event-free survival are estimated every month.

The test design is summarized in Figure 8.

As a result of the feasibility part, combined use of pimitespib and enzalutamide at 160 mg (level 1) and 160 mg, respectively, which are recommended doses of each drug administered alone, was found free from dose limiting toxicity and confirmed to have tolerability. The expansion part is currently underway.

## Claims

**1.** An antitumor agent wherein 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof and an antiandrogen agent are administered in combination.

2. The antitumor agent according to claim 1, wherein the antiandrogen agent is one or more members selected from the group consisting of abiraterone, enzalutamide, darolutamide, and apalutamide.

3. The antitumor agent according to claim 1, wherein the antiandrogen agent is enzalutamide.

4. The antitumor agent according to any one of claims 1 to 3 which is an antitumor agent for treating prostate cancer.

5. The antitumor agent according to any one of claims 1 to 3 which is an antitumor agent for treating castration resistant prostate cancer.

6. The antitumor agent according to any one of claims 1 to 5, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to a cancer patient.

7. An antitumor effect enhancer for an antiandrogen agent, comprising 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof as an active ingredient.

8. The antitumor effect enhancer according to claim 7, wherein the antiandrogen agent is one or more members selected from the group consisting of abiraterone, enzalutamide, darolutamide, and apalutamide.

9. The antitumor effect enhancer according to claim 7, wherein the antiandrogen agent is enzalutamide.

10. The antitumor effect enhancer according to any one of claims 7 to 9 which is an antitumor agent for treating prostate cancer.

11. The antitumor effect enhancer according to any one of claims 7 to 9 which is an antitumor agent for treating castration resistant prostate cancer.

12. The antitumor effect enhancer according to any one of claims 7 to 11, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to a cancer patient.

13. An antitumor agent comprising 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof as an active ingredient, wherein the antitumor agent is administered in combination with an antiandrogen agent.

14. The antitumor agent according to claim 13, wherein the antiandrogen agent is one or more members selected from the group consisting of abiraterone, enzalutamide, darolutamide, and apalutamide.

15. The antitumor agent according to claim 13, wherein the antiandrogen agent is enzalutamide.

16. The antitumor agent according to any one of claims 13 to 15 which is an antitumor agent for treating prostate cancer.

17. The antitumor agent according to any one of claims 13 to 15 which is an antitumor agent for treating castration resistant prostate cancer.

18. The antitumor agent according to any one of claims 13 to 17, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to a cancer patient.

19. An antitumor agent comprising a combination of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof and an antiandrogen agent.

20. The antitumor agent according to claim 19, wherein the antiandrogen agent is one or more members selected from the group consisting of abiraterone, enzalutamide, darolutamide, and apalutamide.

**21.** The antitumor agent according to claim 19, wherein the antiandrogen agent is enzalutamide.

**22.** The antitumor agent according to any one of claims 19 to 21 which is an antitumor agent for treating prostate cancer.

**23.** The antitumor agent according to any one of claims 19 to 21 which is an antitumor agent for treating castration resistant prostate cancer.

**24.** The antitumor agent according to any one of claims 19 to 23, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to a cancer patient.

**25.** A method for treating a cancer, comprising the step of administering a therapeutically effective amount of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof and a therapeutically effective amount of an antiandrogen agent to a cancer patient.

**26.** The method for treating a cancer according to claim 25, wherein the antiandrogen agent is one or more members selected from the group consisting of abiraterone, enzalutamide, darolutamide, and apalutamide.

**27.** The method for treating a cancer according to claim 25, wherein the antiandrogen agent is enzalutamide.

**28.** The method for treating a cancer according to any one of claims 25 to 27, wherein the cancer is prostate cancer.

**29.** The method for treating a cancer according to any one of claims 25 to 27, wherein the cancer is castration resistant prostate cancer.

**30.** The method for treating a cancer according to any one of claims 25 to 29, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to the cancer patient.

**31.** Use of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof for the treatment of a cancer, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof is administered in combination with an antiandrogen agent to a cancer patient.

**32.** The use according to claim 31, wherein the antiandrogen agent is one or more members selected from the group consisting of abiraterone, enzalutamide, darolutamide, and apalutamide.

**33.** The use according to claim 31, wherein the antiandrogen agent is enzalutamide.

**34.** The use according to any one of claims 31 to 33, wherein the cancer is prostate cancer.

**35.** The use according to any one of claims 31 to 33, wherein the cancer is castration resistant prostate cancer.

**36.** The use according to any one of claims 31 to 35, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to the cancer patient.

**37.** Use of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof for the production of an antitumor agent, wherein the antitumor agent is administered in combination with an antiandrogen agent to a cancer patient.

**38.** The use according to claim 37, wherein the antiandrogen agent is one or more members selected from the group consisting of abiraterone, enzalutamide, darolutamide, and apalutamide.

**39.** The use according to claim 37, wherein the antiandrogen agent is enzalutamide.

**40.** The use according to any one of claims 37 to 39, wherein the cancer is prostate cancer.

**41.** The use according to any one of claims 37 to 39, wherein the cancer is castration resistant prostate cancer.

**42.** The use according to any one of claims 37 to 41, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to the cancer patient.

**43.** 3-Ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a salt thereof for use in the treatment of a cancer, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof is administered in combination with an antiandrogen agent to a cancer patient.

**44.** The 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof according to claim 43, wherein the antiandrogen agent is one or more members selected from the group consisting of abiraterone, enzalutamide, darolutamide, and apalutamide.

**45.** The 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof according to claim 43, wherein the antiandrogen agent is enzalutamide.

**46.** The 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof according to any one of claims 43 to 45, wherein the cancer is prostate cancer.

**47.** The 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof according to any one of claims 43 to 45, wherein the cancer is castration resistant prostate cancer.

**48.** The 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof according to any one of claims 43 to 46, wherein the 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or the salt thereof and the antiandrogen agent are administered concurrently or separately in a staggered manner to the cancer patient.

Fig. 1

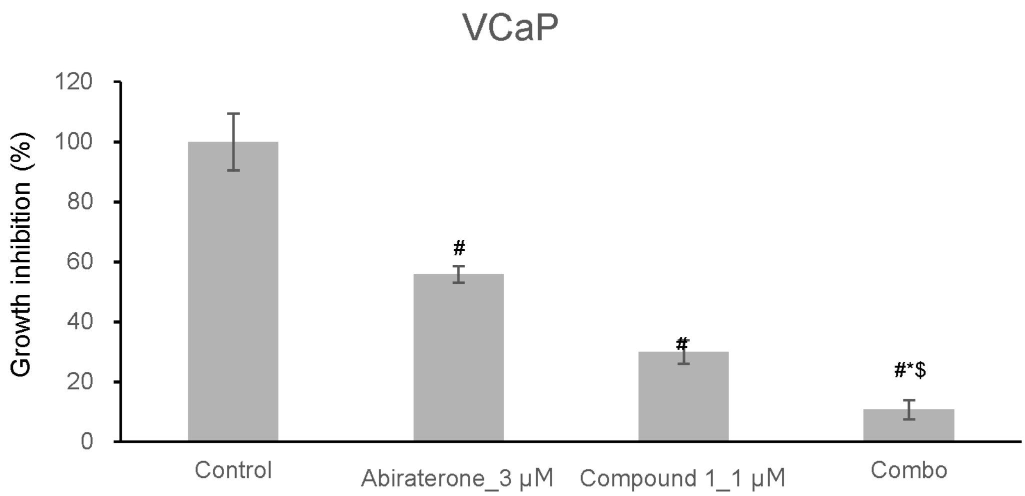


#: $p<0.05$ with Aspin-Welch's *t*-test as compared with the Control group.

*: $p<0.05$ with Aspin-Welch's *t*-test as compared with the Abiraterone group.

$: $p<0.05$ with Aspin-Welch's *t*-test as compared with the Compound 1 control group.

Data are presented as mean ± standard error.

Fig. 2

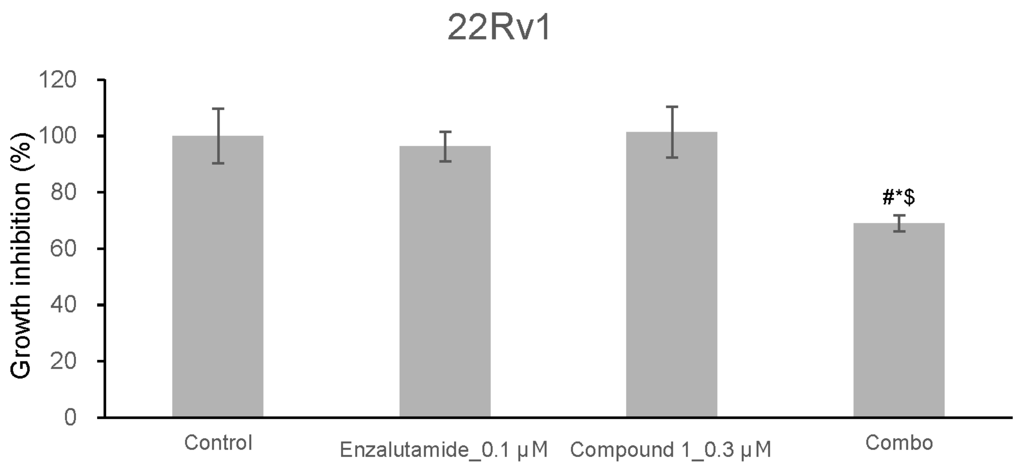


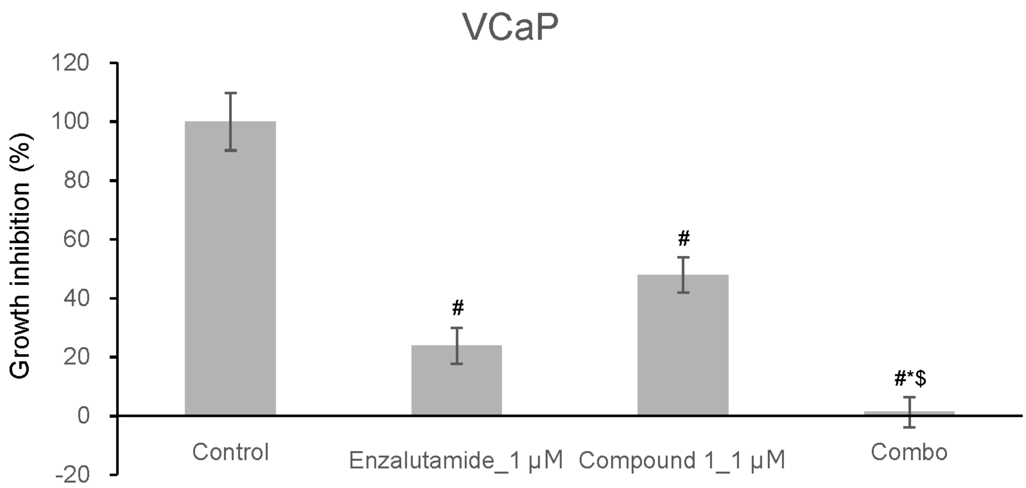


#: $p<0.05$ with Aspin-Welch's *t*-test as compared with the Control group.

*: $p<0.05$ with Aspin-Welch's *t*-test as compared with the Enzalutamide group.

$: $p<0.05$ with Aspin-Welch's *t*-test as compared with the Compound 1 group.

Data are presented as mean ± standard error.

Fig. 3-1

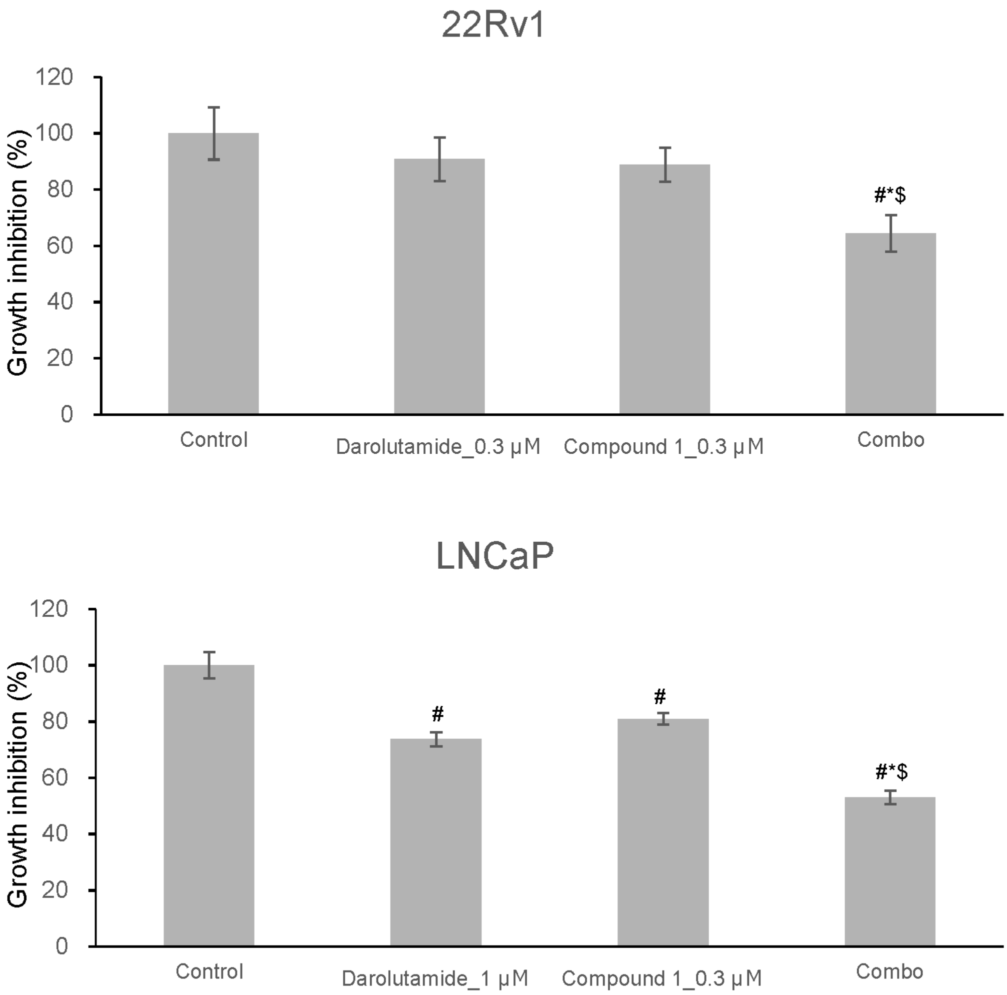
22Rv1
Growth inhibition (%)
120
100
80
60
40
20
0
Control
Darolutamide_0.3 µM
Compound 1_0.3 µM
Combo
#*$
LNCaP
Growth inhibition (%)
120
100
80
60
40
20
0
Control
Darolutamide_1 µM
Compound 1_0.3 µM
Combo
#
#
#*$

Fig. 3-2

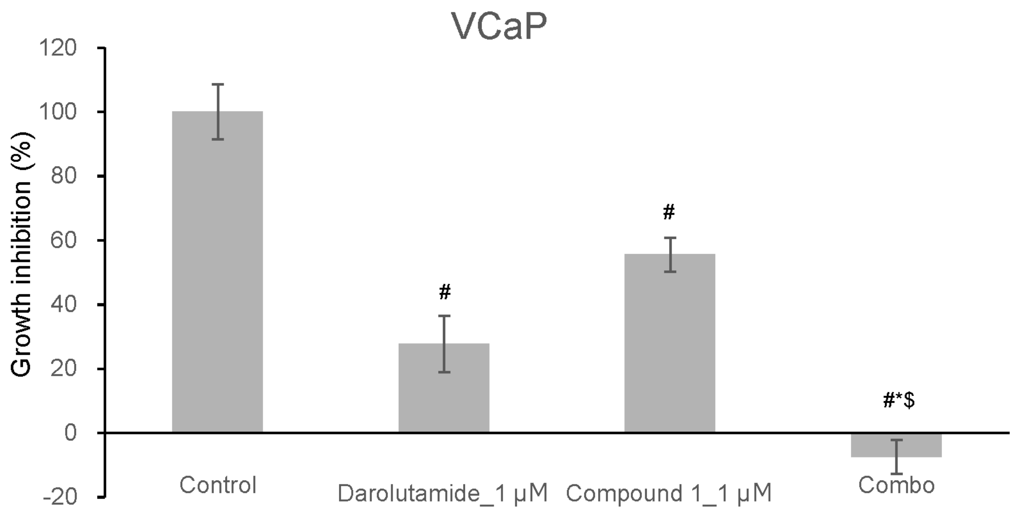


#: p<0.05 with Aspin-Welch's *t*-test as compared with the Control group.

*: p<0.05 with Aspin-Welch's *t*-test as compared with the Darolutamide group.

$: p<0.05 with Aspin-Welch's *t*-test as compared with the Compound 1 group.

Data are presented as mean ± standard error.

Fig. 4

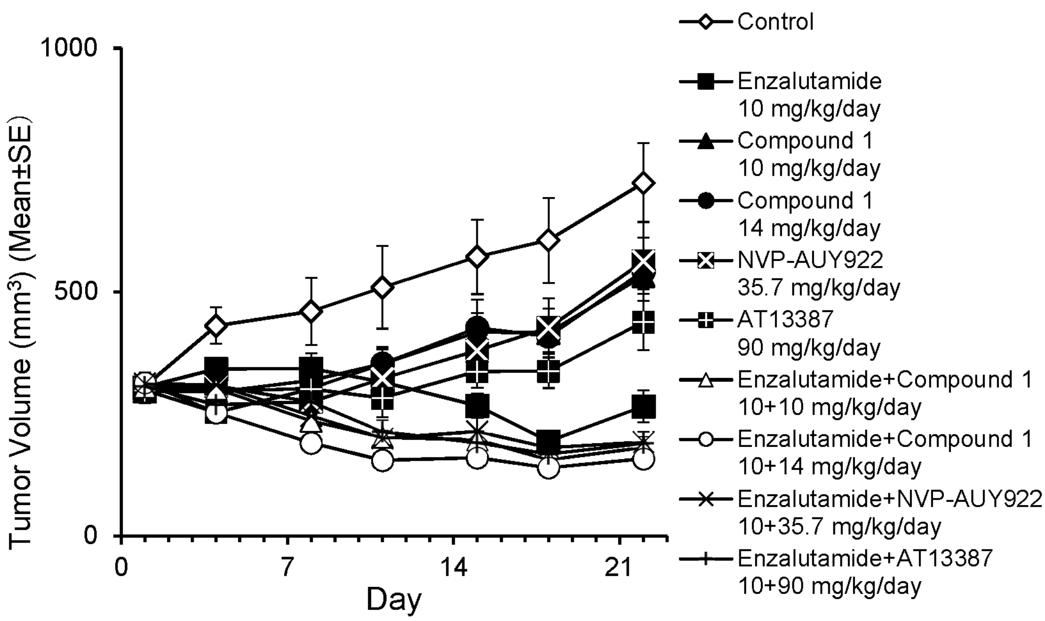

Fig. 5

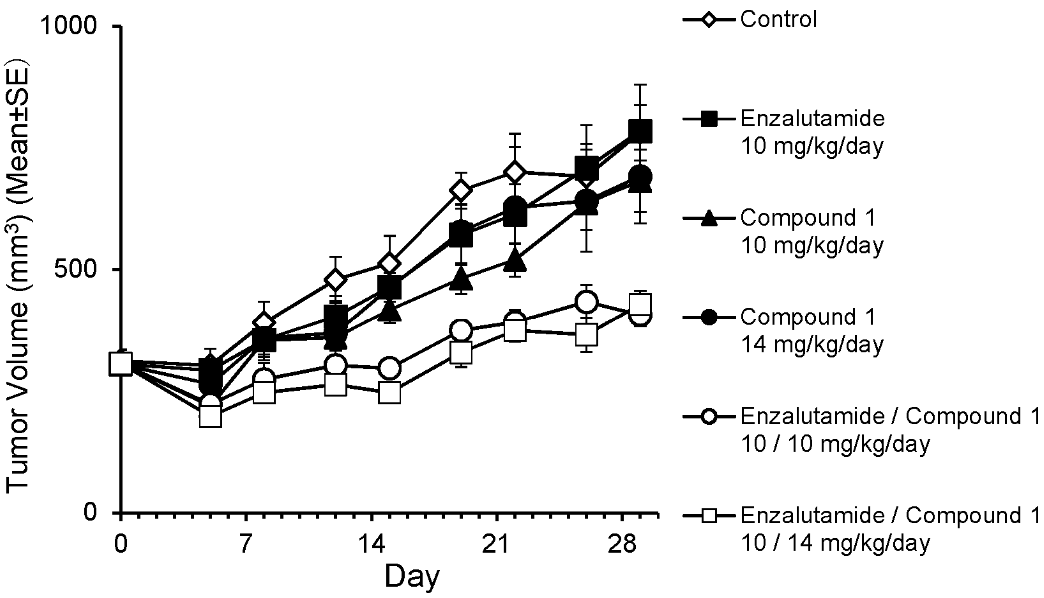
Control
Enzalutamide
10 mg/kg/day
Compound 1
10 mg/kg/day
Compound 1
14 mg/kg/day
Enzalutamide / Compound 1
10 / 10 mg/kg/day
Enzalutamide / Compound 1
10 / 14 mg/kg/day
Tumor Volume ($mm^3$) (Mean±SE)
1000
500
0
0
7
14
21
28
Day

Fig. 6

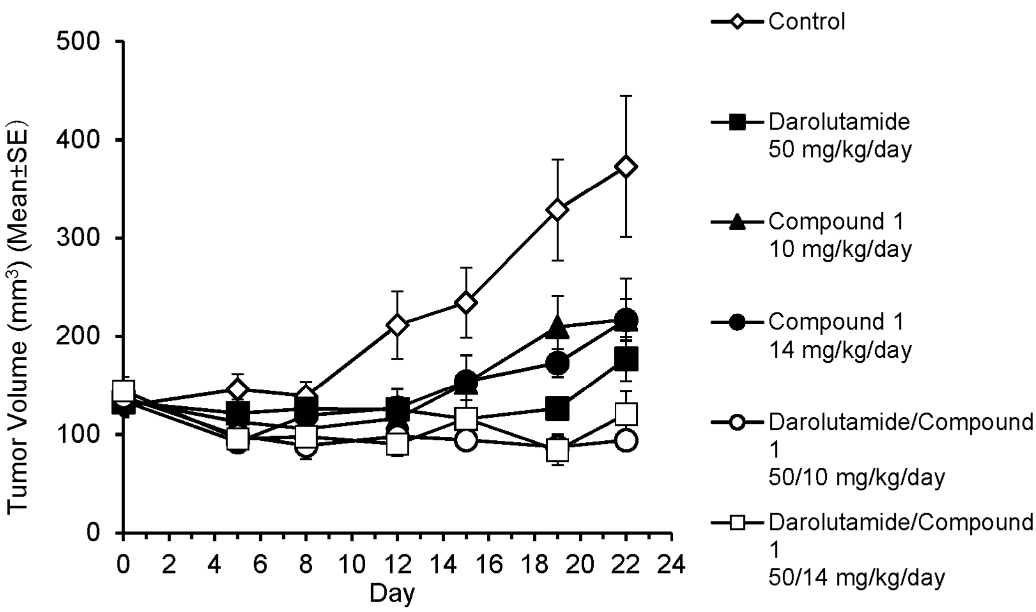
Control
Darolutamide
50 mg/kg/day
Compound 1
10 mg/kg/day
Compound 1
14 mg/kg/day
Darolutamide/Compound
1
50/10 mg/kg/day
Darolutamide/Compound
1
50/14 mg/kg/day
Tumor Volume ($mm^3$) (Mean±SE)
500
400
300
200
100
0
0 2 4 6 8 10 12 14 16 18 20 22 24
Day

Fig. 7

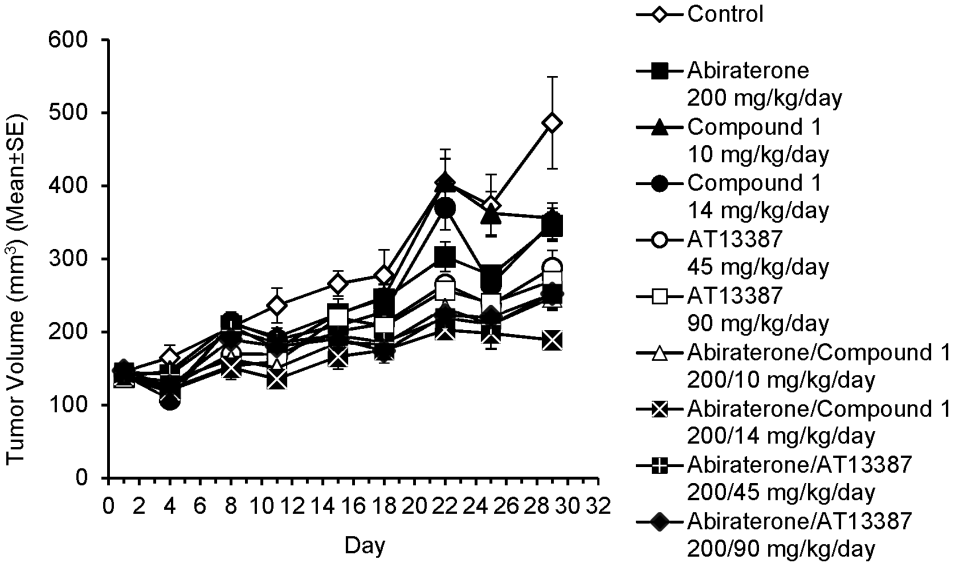


Fig. 8

Summary of test design

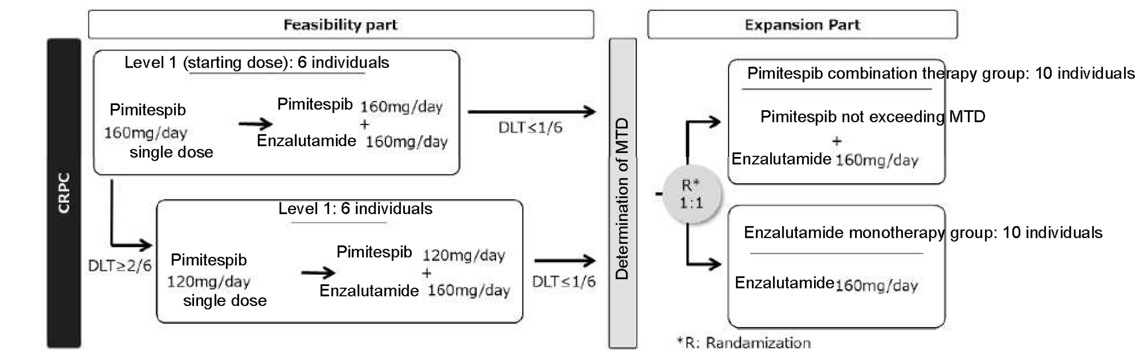

**INTERNATIONAL SEARCH REPORT**

International application No. **PCT/JP2024/029998**

**A. CLASSIFICATION OF SUBJECT MATTER**

***A61K 31/437***(2006.01)i; ***A61K 31/568***(2006.01)i; ***A61K 31/4155***(2006.01)i; ***A61K 31/4439***(2006.01)i; ***A61K 45/00***(2006.01)i; ***A61P 5/28***(2006.01)i; ***A61P 13/08***(2006.01)i; ***A61P 35/00***(2006.01)i; ***A61P 43/00***(2006.01)i

FI: A61K31/437; A61P13/08; A61P35/00; A61P43/00 105; A61P43/00 121; A61P5/28; A61K31/568; A61K31/4155; A61K45/00; A61K31/4439

According to International Patent Classification (IPC) or to both national classification and IPC

**B. FIELDS SEARCHED**

Minimum documentation searched (classification system followed by classification symbols)

A61K31/437; A61K31/568; A61K31/4155; A61K31/4439; A61K45/00; A61P5/28; A61P13/08; A61P35/00; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | WO 2011/004610 A1 (TAIHO PHARMACEUTICAL CO., LTD.) 13 January 2011 (2011-01-13)<br>claims, paragraphs [0006]-[0007], example 102 | 43-48<br><br>1-48 |
| X<br><br>Y | WO 2015/046498 A1 (TAIHO PHARMACEUTICAL CO., LTD.) 02 April 2015 (2015-04-02)<br>claims, paragraphs [0002]-[0007], examples | 43-48<br><br>1-48 |
| Y | CHEN, L. et al., Cotargeting HSP90 and its client proteins for treatment of prostate cancer, Molecular Cancer Therapeutics, 2016, vol. 15, no. 9, pp. 2107-2118<br>abstract, page 2109, left column, sixth paragraph to right column, first paragraph, page 2112, right column, first paragraph to page 2113, left column, first paragraph, fig. 1, 4 | 1-48 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 October 2024** | **15 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/029998**

**C. DOCUMENTS CONSIDERED TO BE RELEVANT**

| Category* | Citation of document, with indication, where appropriate, of the relevant passages | Relevant to claim No. |
|---|---|---|
| Y | CENTENERA, M.M. et al., Co-targeting AR and HSP90 suppresses prostate cancer cell growth and prevents resistance mechanisms, Endocrine-Related Cancer, 2015, vol. 22, no. 5, pp. 805-818 abstract, page 808, right column, fourth paragraph to page 810, left column, first paragraph, page 813, left column, second paragraph to right column, first paragraph, page 815, right column, fourth paragraph to page 816, left column, first paragraph, fig. 1, 5 | 1-48 |
| Y | WO 2008/108386 A1 (KYOUWA HAKKOU KIRINN CO., LTD.) 12 September 2008 (2008-09-12) claims, paragraphs [0057], [0060], [0099] | 1-48 |
| Y | JP 2021-502973 A (INSTITUT REGIONAL DU CANCER DE MONTPELLIER) 04 February 2021 (2021-02-04) claims, examples | 1-48 |

Form PCT/ISA/210 (second sheet) (July 2022)

**INTERNATIONAL SEARCH REPORT**
**Information on patent family members**

International application No.
**PCT/JP2024/029998**

| Patent document cited in search report | Publication date (day/month/year) | Patent family member(s) | Publication date (day/month/year) |
|---|---|---|---|
| WO 2011/004610 A1 | 13 January 2011 | US 2012/0108589 A1<br>claims, paragraphs [0012]-[0013], example 102<br>EP 2452940 A1 | |
| WO 2015/046498 A1 | 02 April 2015 | US 2016/0228417 A1<br>claims, paragraphs [0002]-[0013], examples<br>EP 3053578 A4 | |
| WO 2008/108386 A1 | 12 September 2008 | US 2010/0098690 A1<br>claims, paragraphs [0069], [0075], [0132]<br>EP 2133094 A1 | |
| JP 2021-502973 A | 04 February 2021 | US 2020/0390788 A1<br>claims, examples<br>WO 2019/096824 A1<br>EP 3709988 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011004610 A
- WO 2015046498 A
- WO 2019004417 A
- WO 2019054465 A
- WO 2019221086 A
- WO 2021025065 A


### Non-patent literature cited in the description


- *J Clin Oncol.*, 2012, vol. 30 (6), 644-646
- *Oncotarget.*, 2016, vol. 7 (39), 64447-64470
- *Nat Med.*, 2016, vol. 22 (4), 369-378
- *Lancet Oncol.*, 2019, vol. 20 (12), 1730-1739
- *Cancer Med.*, 2016, vol. 5 (2), 182-191
- *Nat Rev Cancer.*, 2005, vol. 5 (10), 761-772
- *Trends Mol Med.*, 2004, vol. 10 (6), 283-290
- *Endocr Relat Cancer.*, 2015, vol. 22 (5), 805-818
- *Clin Cancer Res.*, 2019, vol. 25 (15), 4624-4633
- *Clin Cancer Res*, 2001, vol. 7, 2941-8